# EUROPÄISCHE PATENTSCHRIFT

(11) **EP 1 066 267 B1**
(45) Veröffentlichungstag und Bekanntmachung des Hinweises auf die Patenterteilung: **12.05.2004**
(21) Anmeldenummer: 98956921.5
(22) Anmeldetag: 17.11.1998
(51) Int. Cl.: C07D 239/54, A01N 43/54, C07D 401/12, C07D 409/12

(54) **SUBSTITUIERTE PHENYLURACILE**
SUBSTITUTED PHENYL URACILS
PHENYLURACILES SUBSTITUEES

(30) Priorität: 28.11.1997 DE 19752748
(43) Veröffentlichungstag der Anmeldung: 10.01.2001
(73) Patentinhaber: Bayer CropScience AG, 40789 Monheim (DE)
(72) Erfinder: ANDREE, Roland, D-40764 Langenfeld (DE); DREWES, Mark, Wilhelm, D-40764 Langenfeld (DE); DOLLINGER, Markus, Overland Park, KS 66213 (US); WETCHOLOWSKY, Ingo, D-51371 Leverkusen (DE); MYERS, Randy, Allen, D-40489 Düsseldorf (DE)
(86) Internationale Anmeldenummer: PCT/EP1998/007342
(87) Internationale Veröffentlichungsnummer: WO 1999/028302

(56) Entgegenhaltungen:
- EP-A- 0 795 550
- WO-A-95/17096
- WO-A-98/39304
- WO-A-98/46592
- WO-A-99/01440
- DE-A- 4 200 259
- US-A- 5 399 543

## Beschreibung

Die Erfindung betrifft neue substituierte Phenyluracile, Verfahren zu ihrer Herstellung und ihre Verwendung als Herbizide.

Eine große Zahl von substituierten Aryluracilen ist bereits aus der (Patent-)Literatur bekannt (vgl. JP-A-05 202 031, JP-A-05 039 272, US-A-5 344 812, US-A-5 399 543, WO-A-95/17096). Diese Verbindungen haben jedoch bisher keine besondere Bedeutung erlangt.

Es wurden nun neue substituierte Phenyluracile der allgemeinen Formel (I) gefunden, 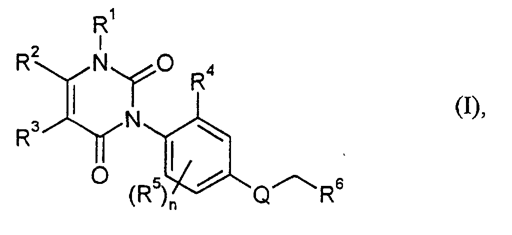 in welcher
- n: für die Zahlen 0, 1, 2 oder 3 steht,
- Q: für O, S, SO, SO₂, NH oder N(C₁-C₄-Alkyl) steht,
- R¹: für Wasserstoff, Amino oder gegebenenfalls durch Cyano, Halogen oder C₁-C₄-Alkoxy substituiertes Alkyl mit 1 bis 4 Kohlenstoffatomen steht,
- R²: für Carboxy, Cyano, Carbamoyl, Thiocarbamoyl oder für jeweils gegebenenfalls durch Cyano, Halogen oder C₁-C₄-Alkoxy substituiertes Alkyl oder Alkoxycarbonyl mit jeweils 1 bis 4 Kohlenstoffatomen steht,
- R³: für Wasserstoff, Halogen oder gegebenenfalls durch Halogen substituiertes Alkyl mit 1 bis 4 Kohlenstoffatomen steht,
- R⁴: für Amino oder für eine der nachstehenden Gruppierungen -NH-CO-R⁷ oder -N(CO-R⁷)₂ steht,
- R⁵: für Nitro, Amino, Hydroxy, Mercapto, Carboxy, Cyano, Carbamoyl, Thiocarbamoyl, Sulfo, Chlorsulfonyl, Aminosulfonyl, Halogen, oder für jeweils gegebenenfalls durch Cyano, Halogen oder C₁-C₄-Alkoxy substituiertes Alkyl, Alkoxy, Alkylthio, Alkylsulfinyl, Alkylsulfonyl, Alkylamino, Alkylcarbonyl, Alkoxycarbonyl, Alkylaminocarbonyl, Alkylcarbonylamino, Alkoxycarbonylamino, Alkylsulfonylamino oder Bis-alkylsulfonyl-amino mit jeweils 1 bis 6 Kohlenstoffatomen in den Alkylgruppen steht,
- R⁶: für jeweils gegebenenfalls durch Nitro, Cyano, Carboxy, Carbamoyl, Thiocarbamoyl, Sulfo, Chlorsulfonyl, Aminosulfonyl, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl oder C₁-C₄-Alkoxycarbonyl substituiertes Phenyl, Naphthyl oder Heterocyclyl aus der Reihe Furyl, Tetrahydrofuryl, Benzofuryl, Dihydrobenzofuryl, Dioxolanyl, Dioxanyl, Benzodioxanyl, Pyrrolyl, Pyrazolyl, Imidazolyl, Benzimidazolyl, Triazolyl, Oxazolyl, Isoxazolyl, Benzoxazolyl, Thiazolyl, Isothiazolyl, Benzthiazolyl, Pyridinyl, Chinolinyl, Isochinolinyl, Pyrimidinyl, Pyrazinyl, Pyridazinyl, Chinazolinyl, Chinoxalinyl steht, und
- R⁷: für Wasserstoff, für jeweils gegebenenfalls durch Cyano, Halogen oder C₁-C₄-Alkoxy substituiertes Alkyl, Alkoxy, Alkylthio, Alkylamino oder Dialkylamino mit jeweils 1 bis 6 Kohlenstoffatomen in den Alkylgruppen, für jeweils gegebenenfalls durch Cyano oder Halogen substituiertes Alkenyl oder Alkinyl mit jeweils 2 bis 6 Kohlenstoffatomen, für jeweils gegebenenfalls durch Cyano, Halogen oder C₁-C₄-Alkyl substituiertes Cycloalkyl oder Cycloalkylalkyl mit jeweils 3 bis 6 Kohlenstoffatomen in den Cycloalkylgruppen und gegebenenfalls 1 bis 4 Kohlenstoffatomen im Alkylteil, oder für jeweils gegebenenfalls durch Nitro, Cyano, Carboxy, Carbamoyl, Thiocarbamoyl, Sulfo, Chlorsulfonyl, Aminosulfonyl, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl oder C₁-C₄-Alkoxy-carbonyl substituiertes Phenyl steht.

Die Erfindung betrifft insbesondere Verbindungen der Formel (I), in welcher
- n: für die Zahlen 0, 1 oder 2 steht,
- Q: für O, S, SO, SO₂, NH oder N(CH₃) steht,
- R¹: für Wasserstoff, Amino oder jeweils gegebenenfalls durch Cyano, Fluor, Chlor, Methoxy oder Ethoxy substituiertes Methyl, Ethyl, n- oder i-Propyl steht,
- R²: für Carboxy, Cyano, Carbamoyl, Thiocarbamoyl oder für jeweils gegebenenfalls durch Cyano, Fluor, Chlor, Methoxy oder Ethoxy substituiertes Methyl, Ethyl, n- oder i-Propyl, Methoxycarbonyl, Ethoxycarbonyl, n- oder i-Propoxycarbonyl steht,
- R³: für Wasserstoff, Fluor, Chlor, Brom oder für jeweils gegebenenfalls durch Fluor und/oder Chlor substituiertes Methyl, Ethyl, n- oder i-Propyl steht,
- R⁴: für Amino oder für eine der nachstehenden Gruppierungen -NH-CO-R⁷ oder -N(CO-R⁷)₂ steht,
- R⁵: für Nitro, Amino, Hydroxy, Mercapto, Carboxy, Cyano, Carbamoyl, Thiocarbamoyl, Sulfo, Chlorsulfonyl, Aminosulfonyl, Fluor, Chlor, Brom, oder für jeweils gegebenenfalls durch Cyano, Fluor, Chlor, Methoxy oder Ethoxy substituiertes Methyl, Ethyl, n- oder i-Propyl, Methoxy, Ethoxy, n- oder i-Propoxy, Methylthio, Ethylthio, n- oder i-Propylthio, Methylsulfinyl, Ethylsulfinyl, Methylsulfonyl, Ethylsulfonyl, Methylamino, Ethylamino, n- oder i-Propylamino, Acetyl, Propionyl, Methoxycarbonyl, Ethoxycarbonyl, Methylaminocarbonyl, Ethylaminocarbonyl, Acetylamino, Propionylamino, Methoxycarbonylamino, Ethoxycarbonylamino, Methylsulfonylamino, Ethylsulfonylamino, Bis-methylsulfonyl-amino oder Bis-ethylsulfonyl-amino steht,
- R⁶: für jeweils gegebenenfalls durch Nitro, Cyano, Carboxy, Carbamoyl, Thiocarbamoyl, Sulfo, Chlorsulfonyl, Aminosulfonyl, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl oder C₁-C₄-Alkoxycarbonyl substituiertes Phenyl, Naphthyl oder Heterocyclyl aus der Reihe Furyl, Tetrahydrofuryl, Benzofuryl, Dihydrobenzofuryl, Dioxolanyl, Dioxanyl, Benzodioxanyl, Pyrrolyl, Pyrazolyl, Imidazolyl, Benzimidazolyl, Triazolyl, Oxazolyl, Isoxazolyl, Benzoxazolyl, Thiazolyl, Isothiazolyl, Benzthiazolyl, Pyridinyl, Chinolinyl, Isochinolinyl, Pyrimidinyl, Pyrazinyl, Pyridazinyl, Chinazolinyl, Chinoxalinyl steht, und
- R⁷: für Wasserstoff, für jeweils gegebenenfalls durch Cyano, Halogen oder C₁-C₄-Alkoxy substituiertes Alkyl, Alkoxy, Alkylthio, Alkylamino oder Dialkylamino mit jeweils 1 bis 6 Kohlenstoffatomen in den Alkylgruppen, für jeweils gegebenenfalls durch Cyano oder Halogen substituiertes Alkenyl oder Alkinyl mit jeweils 2 bis 6 Kohlenstoffatomen, für jeweils gegebenenfalls durch Cyano, Halogen oder C₁-C₄-Alkyl substituiertes Cycloalkyl oder Cycloalkylalkyl mit jeweils 3 bis 6 Kohlenstoffatomen in den Cycloalkylgruppen und gegebenenfalls 1 bis 4 Kohlenstoffatomen im Alkylteil, oder für jeweils gegebenenfalls durch Nitro, Cyano, Carboxy, Carbamoyl, Thiocarbamoyl, Sulfo, Chlorsulfonyl, Aminosulfonyl, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl oder C₁-C₄-Alkoxy-carbonyl substituiertes Phenyl, steht.

Ganz bevorzugt sind Verbindungen der Formel (I),
in welcher
- n: für die Zahl 0 steht,
- Q: für O steht,
- R¹: für Wasserstoff und noch bevorzugter für Methyl steht,
- R²: für Trifluormethyl steht,
- R³: für Wasserstoff steht,
- R⁴: für eine Gruppe, wie in Tabelle 1 angegeben, steht, und
- R⁶: für in para-Stellung durch Chlor substituiertes Phenyl steht.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- oder Zwischenprodukte. Diese Restedefinitionen können untereinander, also auch zwischen den angegebenen bevorzugten Bereichen beliebig kombiniert werden.

Beispiele für die erfindungsgemäßen Verbindungen der allgemeinen Formel (I) sind in den nachstehenden Gruppen aufgeführt.

### Gruppe 1

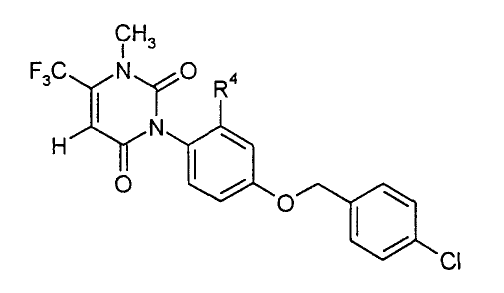

R⁴ hat dabei die in der nachstehenden Auflistung angegebenen Bedeutungen: NO₂, NH₂, -NH-CO-CH₃, -NH-CO-C₂H₅, -NH-CO-C₃H₇-n, -NH-CO-C₃H₇-i, -NH-CO-C₄H₉-n, -NH-CO-C₄H₉-i, -NH-CO-C₄H₉-s, -NH-CO-C₄H₉-t, -NH-CO-CF₃, -NH-CO-CHCl₂, -NH-CO-CH₂OCH₃, -NH-CO-Cyclopropyl, -NH-CO-Phenyl, -NH-CO-(3-Fluor-phenyl), -NH-CO-(4-Fluor-phenyl), -NH-CO-(3-Chlor-phenyl), -NH-CO-(4-Chlor-phenyl), -NH-CO-(3-Methyl-phenyl), -NH-CO-(4-Methyl-phenyl), -NH-CO-(3-Trifluormethyl-phenyl), -NH-CO-(3-Trifluormethylphenyl), -NH-CO-(3-Methoxy-phenyl), -NH-CO-(4-Methoxy-phenyl), -NH-CO-(4-Trifluormethoxy-phenyl), -NH-CO-(2-Furyl), -NH-CO-(3-Furyl), -NH-CO-(2-Thienyl), -NH-CO-(3-Thienyl), -N(CO-CH₃)2, -N(CO-C₂H₅)₂, -N(CO-CF₃)₂.

### Gruppe 2

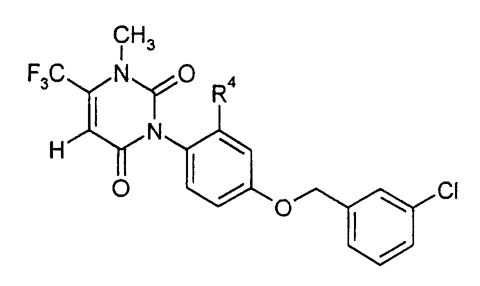

R⁴ hat dabei die oben in Gruppe 1 angegebenen Bedeutungen.

### Gruppe 3

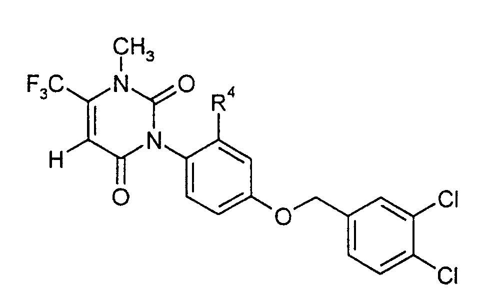

R⁴ hat dabei die oben in Gruppe 1 angegebenen Bedeutungen.

### Gruppe 4

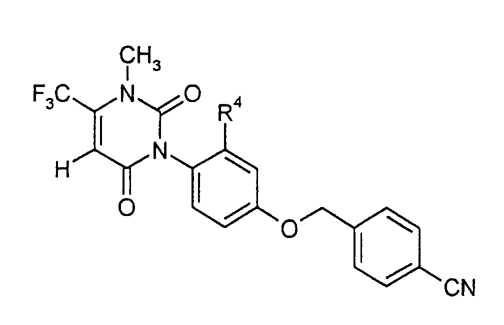

R⁴ hat dabei die oben in Gruppe 1 angegebenen Bedeutungen.

### Gruppe 5

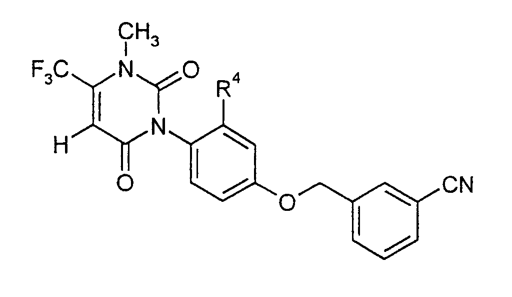

R⁴ hat dabei die oben in Gruppe 1 angegebenen Bedeutungen.

### Gruppe 6

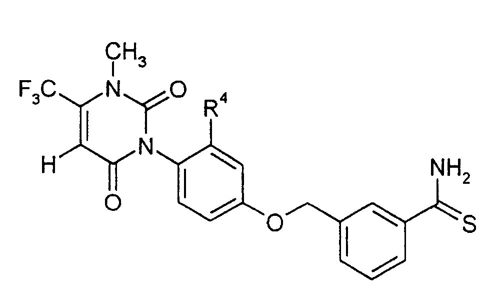

R⁴ hat dabei die oben in Gruppe 1 angegebenen Bedeutungen.

### Gruppe 7

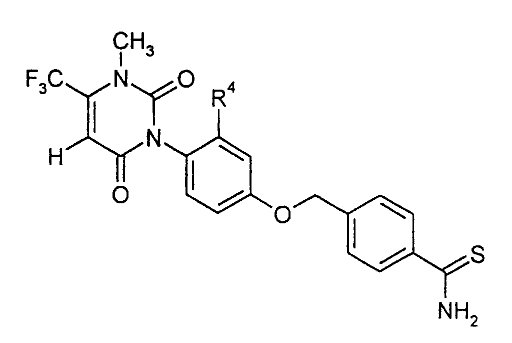

R⁴ hat dabei die oben in Gruppe 1 angegebenen Bedeutungen.

### Gruppe 8

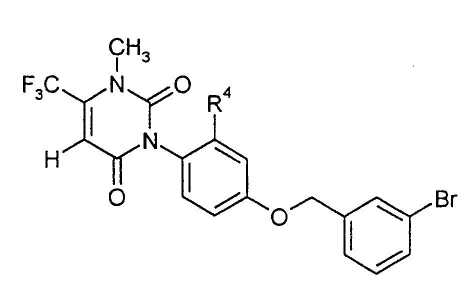

R⁴ hat dabei die oben in Gruppe 1 angegebenen Bedeutungen.

### Gruppe 9

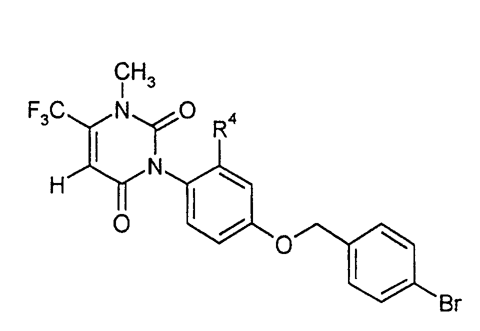

R⁴ hat dabei die oben in Gruppe 1 angegebenen Bedeutungen.

### Gruppe 10

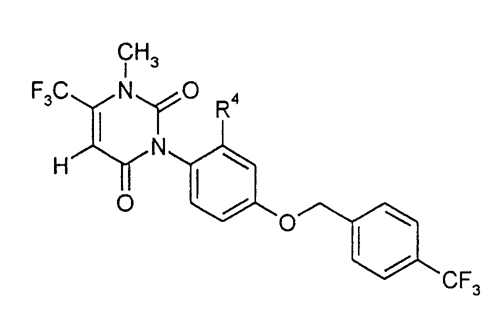

R⁴ hat dabei die oben in Gruppe 1 angegebenen Bedeutungen.

### Gruppe 11

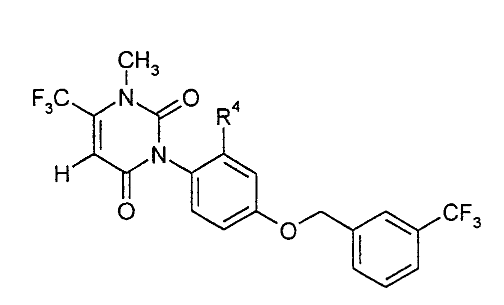

R⁴ hat dabei die oben in Gruppe 1 angegebenen Bedeutungen.

### Gruppe 12

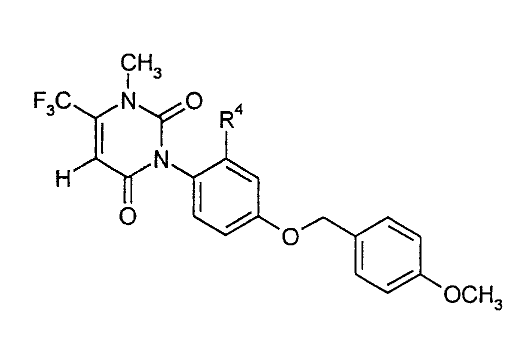

R⁴ hat dabei die oben in Gruppe 1 angegebenen Bedeutungen.

### Gruppe 13

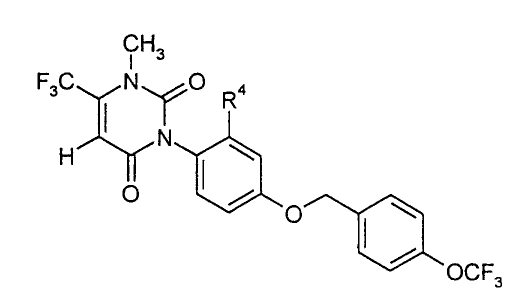

R⁴ hat dabei die oben in Gruppe 1 angegebenen Bedeutungen.

### Gruppe 14

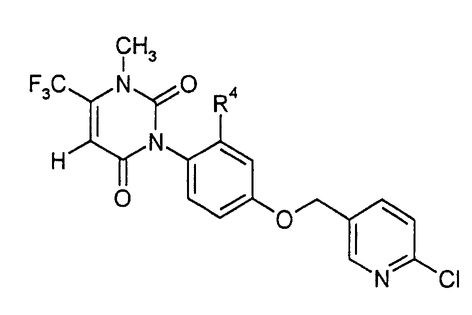

R⁴ hat dabei die oben in Gruppe 1 angegebenen Bedeutungen.

### Gruppe 15

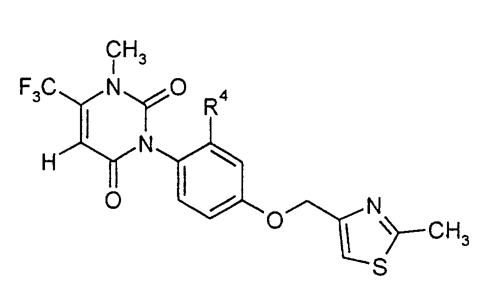

R⁴ hat dabei die oben in Gruppe 1 angegebenen Bedeutungen.

### Gruppe 16

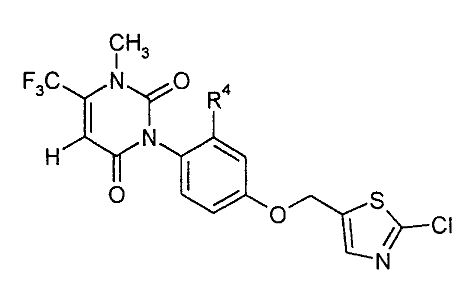

R⁴ hat dabei die oben in Gruppe 1 angegebenen Bedeutungen.

Die neuen substituierten Phenyluracile der allgemeinen Formel (I) zeichnen sich durch starke und selektive herbizide Wirksamkeit aus.

Man erhält die neuen substituierten Phenyluracile der allgemeinen Formel (I), wenn man
(a) Aminoalkensäureester der allgemeinen Formel (II) 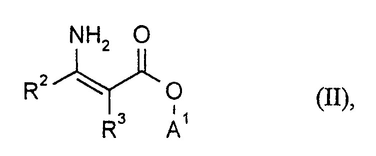 in welcher
   - R² und R³: die oben angegebenen Bedeutungen haben und
   - A¹: für Alkyl, Aryl oder Arylalkyl steht,
   mit substituierten Phenylurethanen (Phenylcarbamaten) der allgemeinen Formel (III) 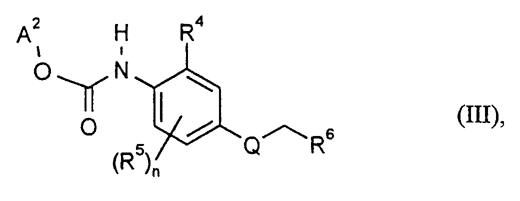 in welcher
   - n, Q, R⁴, R⁵ und R⁶: die oben angegebenen Bedeutungen haben und
   - A²: für Alkyl, Aryl oder Arylalkyl steht,
   gegebenenfalls in Gegenwart eines Reaktionshilfsmittels und gegebenenfalls in Gegenwart eines Verdünnungsmittels umsetzt,
   oder wenn man
(b) substituierte Phenyluracile der allgemeinen Formel (Ia), 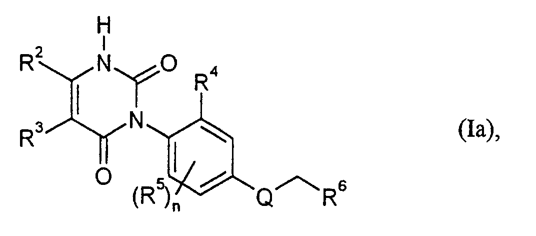 in welcher
   - n, Q, R², R³, R⁴, R⁵ und R⁶: die oben angegebenen Bedeutungen haben,
   mit 1-Aminooxy-2,4-dinitro-benzol oder mit Alkylierungsmitteln der allgemeinen Formel (IV)

   X¹-A³ (IV),

   in welcher
   - A³: für gegebenenfalls substituiertes Alkyl steht und
   - X¹: für Halogen oder die Gruppierung -O-SO₂-O-A³ steht,
   gegebenenfalls in Gegenwart eines Reaktionshilfsmittels und gegebenenfalls in Gegenwart eines Verdünnungsmittels umsetzt, oder wenn man
(c) substituierte Phenyluracile der allgemeinen Formel (Ib), 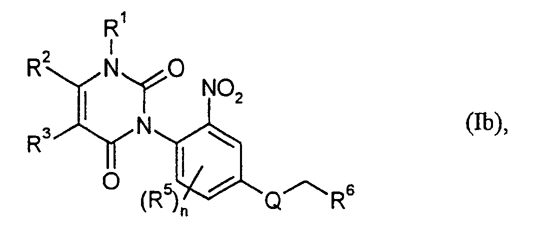 in welcher
   - n, Q, R¹, R², R³, R⁵ und R⁶: die oben angegebenen Bedeutungen haben,
   mit Hydrierungsmitteln gegebenenfalls in Gegenwart von Reaktionshilfsmitteln und gegebenenfalls in Gegenwart von Verdünnungsmitteln umsetzt,
   oder wenn man
(d) substituierte Phenyluracile der allgemeinen Formel (Ic), 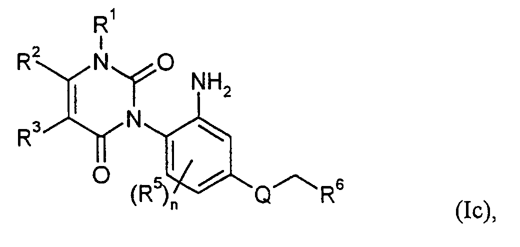 in welcher
   - n, Q, R¹, R², R³, R⁵ und R⁶: die oben angegebenen Bedeutungen haben,
   mit Acylierungsmitteln der allgemeinen Formel (V)

   X²-CO-R⁷ (V)

   in welcher
   - R⁷: die oben angegebene Bedeutung hat und
   - X²: für Halogen oder die die Gruppierung -O-CO-R⁷ steht,
   gegebenenfalls in Gegenwart von Reaktionshilfsmitteln und gegebenenfalls in Gegenwart von Verdünnungsmitteln umsetzt.

Verwendet man beispielsweise 3-Amino-2,4,4,4-tetrafluor-crotonsäure-methylester und N-[4-(2-Chlor-phenylmethoxy)-2-nitro-phenyl]-O-methyl-carbamat als Ausgangsstoffe, so kann der Reaktionsablauf beim erfindungsgemäßen Verfahren (a) durch das folgende Formelschema skizziert werden:

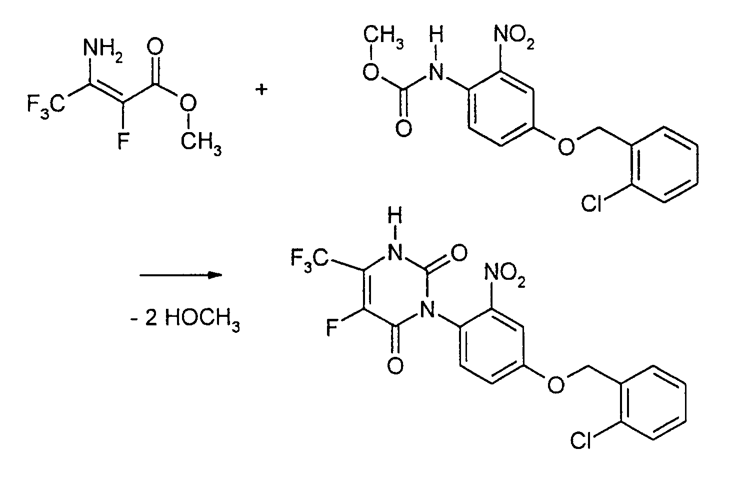

Verwendet man beispielsweise 1-[4-(3-methyl-phenylmethoxy)-2-nitro-phenyl]-5-chlor-4-chlordifluormethyl-3,6-dihydro-2,6-dioxo-1(2H)-pyrimidin und Ethylbromid als Ausgangsstoffe, so kann der Reaktionsablauf beim erfindungsgemäßen Verfahren (b) durch das folgende Formelschema skizziert werden:

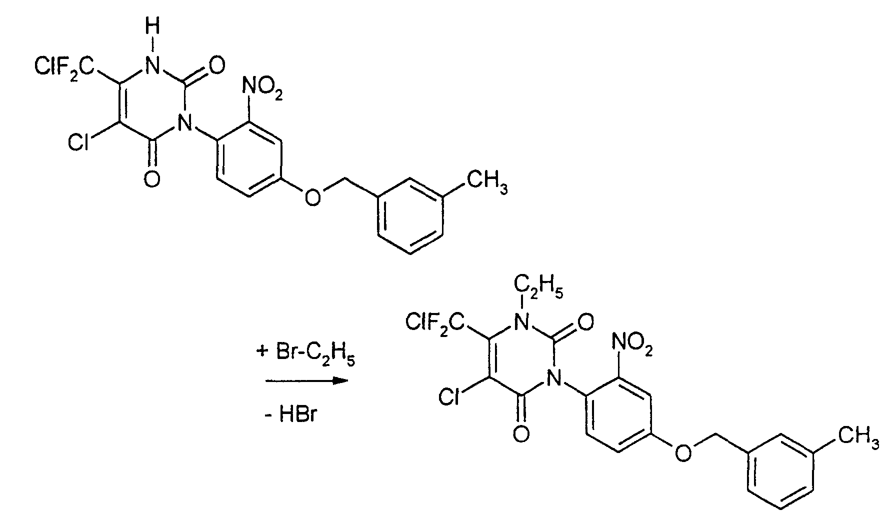

Verwendet man beispielsweise 1-[2-Nitro-4-(2-trifluormethyl-phenylmethoxy)]-3,5-dimethyl-4-trifluormethyl-3,6-dihydro-2,6-dioxo-1(2H)-pyrimidin und Wasserstoff in Gegenwart eines Katalysators, wie z.B. Platin oder Palladium, als Ausgangsstoffe, so kann der Reaktionsablauf beim erfindungsgemäßen Verfahren (c) durch das folgende Formelschema skizziert werden:

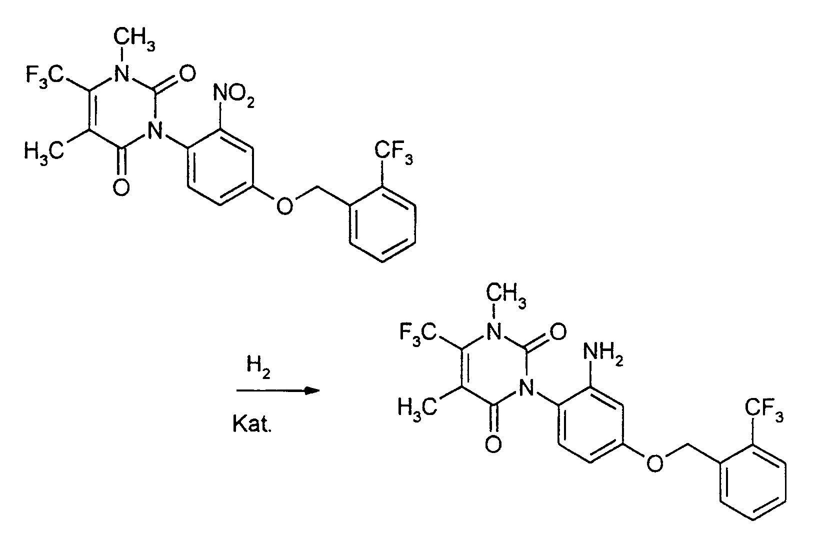

Verwendet man beispielsweise 1-[2-Amino-4-(2-chlor-pyridin-5-ylmethoxy)]-3-methyl-4-trifluormethyl-3,6-dihydro-2,6-dioxo-1(2H)-pyrimidin und Propionsäurechlorid als Ausgangsstoffe, so kann der Reaktionsablauf beim erfindungsgemäßen Verfahren (d) durch das folgende Formelschema skizziert werden:

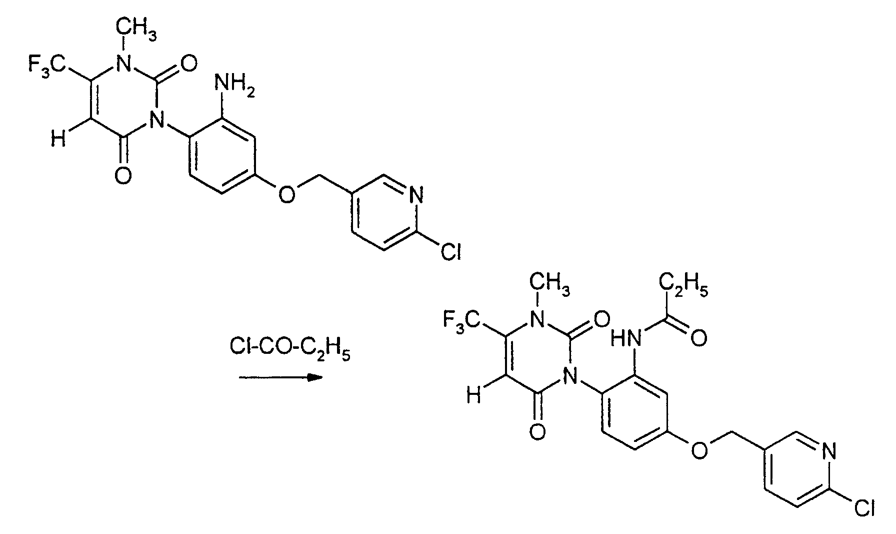

Die beim erfindungsgemäßen Verfahren (a) zur Herstellung von Verbindungen der Formel (I) als Ausgangsstoffe zu verwendenden Aminoalkensäureester sind durch die Formel (II) allgemein definiert. In der Formel (II) haben R² und R³ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R² und R³ angegeben wurden; A¹ steht vorzugsweise für C₁-C₄-Alkyl, Phenyl oder Benzyl, insbesondere für Methyl oder Ethyl.

Die Ausgangsstoffe der allgemeinen Formel (II) sind bekannt und/oder können nach bekannten Verfahren hergestellt werden (vgl. J. Heterocycl. Chem. 9 (1972), 513 bis 522).

Die beim erfindungsgemäßen Verfahren (a) weiter als Ausgangsstoffe zu verwendenden substituierten Phenylurethane sind durch die Formel (III) allgemein definiert. In der Formel (III) haben n, Q, R⁴, R⁵ und R⁶ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für n, Q, R⁴, R⁵ und R⁶ angegeben wurden; A² steht vorzugsweise für C₁-C₄-Alkyl, Phenyl oder Benzyl, insbesondere für Methyl oder Ethyl.

Die Ausgangsstoffe der allgemeinen Formel (III) sind noch nicht aus der Literatur bekannt; sie sind als neue Stoffe auch Gegenstand der vorliegenden Anmeldung.

Man erhält die substituierten Phenylurethane (Phenylcarbamate) der allgemeinen Formel (III), wenn man substituierte Phenylamine der allgemeinen Formel (VI) 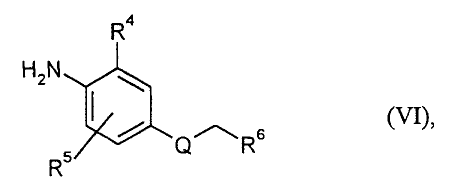 in welcher
- n, Q, R⁴, R⁵ und R⁶: die oben angegebenen Bedeutungen haben,
mit Chlorameisensäureestern der allgemeinen Formel (VII)

A²-O-CO-Cl (VII)

in welcher
- A²: die oben angegebene Bedeutung hat,
gegebenenfalls in Gegenwart eines Säureakzeptors, wie z.B. Pyridin, und gegebenenfalls in Gegenwart eines Verdünnungsmittels, wie z.B. Methylenchlorid, bei Temperaturen zwischen 0°C und 100°C umsetzt (vgl. die Herstellungsbeispiele).

Die als Vorprodukte benötigten substituierte Phenylamine der allgemeinen Formel (VI) sind bekannt und/oder können nach an sich bekannten Verfahren hergestellt werden (vgl. Justus Liebigs Ann. Chem. 733 (1970), 125-140; DE-A-28 42 186; Herstellungsbeispiele).

Die beim erfindungsgemäßen Verfahren (b) zur Herstellung von Verbindungen der Formel (I) als Ausgangsstoffe zu verwendenden substituierten Phenyluracile sind durch die Formel (Ia) allgemein definiert. In der Formel (Ia) haben n, Q, R², R³, R⁴, R⁵ und R⁶ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für n, Q, R², R³, R⁴, R⁵ und R⁶ angegeben wurden.

Die Ausgangsstoffe der allgemeinen Formel (Ia) für Verfahren (b) sind als neue Stoffe auch Gegenstand der vorliegenden Anmeldung; sie können nach dem erfindungsgemäßen Verfahren (a) hergestellt werden.

Die beim erfindungsgemäßen Verfahren (b) weiter als Ausgangsstoffe zu verwendenden Alkylierungsmittel sind durch die Formel (IV) allgemein definiert. In der Formel (IV) stehen vorzugsweise A³ für gegebenenfalls durch Cyano, Halogen oder C₁-C₄-Alkoxy substituiertes Alkyl mit 1 bis 4 Kohlenstoffatomen und X¹ für Chlor, Brom, Iod, Methylsulfonyloxy oder Ethylsulfonyloxy; insbesondere stehen A³ für jeweils gegebenenfalls durch Cyano, Fluor, Chlor, Methoxy oder Ethoxy substituiertes Methyl, Ethyl, n- oder i-Propyl und X¹ für Chlor, Brom, Iod, Methylsulfonyloxy oder Ethylsulfonyloxy.

Die Ausgangsstoffe der Formel (IV) sind bekannte organische Synthesechemikalien.

Die beim erfindungsgemäßen Verfahren (c) zur Herstellung von Verbindungen der Formel (I) als Ausgangsstoffe zu verwendenden substituierten Phenyluracile sind durch die Formel (Ib) allgemein definiert. In der Formel (Ib) haben n, Q, R¹, R², R³, R⁵ und R⁶ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für n, Q, R¹, R², R³, R⁵ und R⁶ angegeben wurden.

Die Ausgangsstoffe der allgemeinen Formel (Ib) für Verfahren (c) sind als neue Stoffe auch Gegenstand der vorliegenden Anmeldung; sie können nach den erfindungsgemäßen Verfahren (a) oder (b) hergestellt werden.

Die beim erfindungsgemäßen Verfahren (d) zur Herstellung von Verbindungen der Formel (I) als Ausgangsstoffe zu verwendenden substituierten Phenyluracile sind durch die Formel (Ic) allgemein definiert. In der Formel (Ic) haben n, Q, R¹, R², R³, R⁵ und R⁶ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für n, Q, R¹, R², R³, R⁵ und R⁶ angegeben wurden.

Die Ausgangsstoffe der allgemeinen Formel (Ic) für Verfahren (d) sind als neue Stoffe auch Gegenstand der vorliegenden Anmeldung; sie können nach den erfindungsgemäßen Verfahren (a), (b) oder (c) hergestellt werden.

Die beim erfindungsgemäßen Verfahren (d) weiter als Ausgangsstoffe zu verwendenden Acylierungsmittel sind durch die Formel (V) allgemein definiert. In der Formel (V) hat R⁷ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für R⁷ angegeben wurde; X² steht vorzugsweise für Fluor, Chlor oder Brom, insbesondere für Chlor.

Die Ausgangsstoffe der allgemeinen Formel (V) sind bekannte organische Synthesechemikalien.

Die erfindungsgemäßen Verfahren zur Herstellung der Verbindungen der allgemeinen Formel (I) werden vorzugsweise unter Verwendung von Verdünnungsmitteln durchgeführt. Als Verdünnungsmittel zur Durchführung der erfindungsgemäßen Verfahren (a) bis (d) kommen neben vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester, Sulfoxide, wie Dimethylsulfoxid, Alkohole, wie Methanol, Ethanol, n- oder i-Propanol, Ethylenglykolmonomethylether, Ethylenglykolmonoethylether, Diethylenglykolmonomethylether, Diethylenglykolmonoethylether, deren Gemische mit Wasser oder reines Wasser.

Als Reaktionshilfsmittel für die erfindungsgemäßen Verfahren (a) (b) und (d) kommen im allgemeinen die üblichen anorganischen oder organischen Basen oder Säureakzeptoren in Betracht. Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kaliumoder Calcium-hydrid, Lithium-, Natrium-, Kalium- oder Calcium-hydroxid, Natriumoder Kalium-methanolat, -ethanolat, -n- oder -i-propanolat, -n-, -i-, -s- oder -tbutanolat; weiterhin auch basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), oder 1,8-Diazabicyclo[5,4,0]-undec-7-en (DBU).

Das erfindungsgemäße Verfahren (c) wird unter Verwendung eines Hydrierungsmittels durchgeführt. Es können hierbei die üblichen zur Reduktion von Nitroverbindungen geeigneten Hydrierungsmittel eingesetzt werden. Hierzu gehören beispielweise Wasserstoff, Hydrazin(-hydrat), Metalle, wie z.B. Eisen, in Gegenwart einer Säure, wie z.B. Salzsäure, Schwefelsäure oder Essigsäure, Sulfide, wie z.B. Natriumsulfid, und Hydridkomplexe, wie z.B. Natriumboranat.

Als Reaktionshilfsmittel - insbesondere bei Verwendung von Wasserstoff oder Hydrazin(-hydrat) als Hydrierungsmittel - können Metalle, wie Platin, Palladium, Eisen, Cobalt, Nickel, und gegebenenfalls zusätzlich Trägermaterialien, wie Kohle oder Kieselgur, verwendet werden.

Die Reaktionstemperaturen können bei der Durchführung der erfindungsgemäßen Verfahren (a) bis (d) in einem größeren Bereich variiert werden. Im allgemeinen arbeitet man bei Temperaturen zwischen 0°C und 200°C, vorzugsweise zwischen 10°C und 150°C.

Die erfindungsgemäße Verfahren werden im allgemeinen unter Normaldruck durchgeführt. Es ist jedoch auch möglich, die erfindungsgemäße Verfahren unter erhöhtem oder vermindertem Druck - im allgemeinen zwischen 0,1 bar und 100 bar - durchzuführen.

Zur Durchführung der erfindungsgemäßen Verfahren werden die Ausgangsstoffe im allgemeinen in angenähert äquimolaren Mengen eingesetzt. Es ist jedoch auch möglich, jeweils eine der Komponenten in einem größeren Überschuß zu verwenden. Die Umsetzung wird im allgemeinen in einem geeigneten Verdünnungsmittel in Gegenwart eines Reaktionshilfsmittels durchgeführt und das Reaktionsgemisch wird im allgemeinen mehrere Stunden bei der erforderlichen Temperatur gerührt. Die Aufarbeitung wird nach üblichen Methoden durchgeführt (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen Kulturen sowohl im Vorauflauf- als auch im Nachauflauf-Verfahren.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Acetochlor, Acifluorfen(-sodium), Aclonifen, Alachlor, Alloxydim(-sodium), Ametryne, Amidochlor, Amidosulfuron, Anilofos, Asulam, Atrazine, Azafenidin, Azimsulfuron, Benazolin(-ethyl), Benfuresate, Bensulfuron(-methyl), Bentazon, Benzofenap, Benzoylprop(-ethyl), Bialaphos, Bifenox, Bispyribac(-sodium), Bromobutide, Bromofenoxim, Bromoxynil, Butachlor, Butroxydim, Butylate, Cafenstrole, Caloxydim, Carbetamide, Carfentrazone(-ethyl), Chlomethoxyfen, Chloramben, Chloridazon, Chlorimuron(-ethyl), Chlornitrofen, Chlorsulfuron, Chlortoluron, Cinmethylin, Cinosulfuron, Clethodim, Clodinafop(-propargyl), Clomazone, Clomeprop, Clopyralid, Clopyrasulfuron(-methyl), Cloransulam(-methyl), Cumyluron, Cyanazine, Cycloate, Cyclosulfamuron, Cycloxydim, Cyhalofop(-butyl), 2,4-D, 2,4-DB, 2,4-DP, Desmedipham, Diallate, Dicamba, Diclofop(-methyl), Diclosulam, Diethatyl(-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epoprodan, EPTC, Esprocarb, Ethalfluralin, Ethametsulfuron(-methyl), Ethofumesate, Ethoxyfen, Ethoxysulfuron, Etobenzanid, Fenoxaprop(-P-ethyl), Flamprop(-isopropyl), Flamprop(-isopropyl-L), Flamprop(-methyl), Flazasulfuron, Fluazifop(-P-butyl), Flumetsulam, Flumiclorac(-pentyl), Flumioxazin, Flumipropyn, Flumetsulam, Fluometuron, Fluorochloridone, Fluoroglycofen(-ethyl), Flupoxam, Flupropacil, Flurpyrsulfuron(-methyl, -sodium), Flurenol(-butyl), Fluridone, Fluroxypyr(-meptyl), Flurprimidol, Flurtamone, Fluthiacet(-methyl), Fluthiamide, Fomesafen, Glufosinate(-ammonium), Glyphosate(-isopropylammonium), Halosafen, Haloxyfop(-ethoxyethyl), Haloxyfop(-P-methyl), Hexazinone, Imazamethabenz(-methyl), Imazamethapyr, Imazamox, Imazapyr, Imazaquin, Imazethapyr, Imazosulfuron, Ioxynil, Isopropalin, Isoproturon, Isouron, Isoxaben, Isoxaflutole, Isoxapyrifop, Lactofen, Lenacil, Linuron, MCPA, MCPP, Mefenacet, Metamitron, Metazachlor, Methabenzthiazuron, Metobenzuron, Metobromuron, (alpha-)Metolachlor, Metosulam, Metoxuron, Metribuzin, Metsulfuron(-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pentoxazone, Phenmedipham, Piperophos, Pretilachlor, Primisulfuron(-methyl), Prometryn, Propachlor, Propanil, Propaquizafop, Propisochlor, Propyzamide, Prosulfocarb, Prosulfuron, Pyraflufen(-ethyl), Pyrazolate, Pyrazosulfuron(-ethyl), Pyrazoxyfen, Pyribenzoxim, Pyributicarb, Pyridate, Pyriminobac(-methyl), Pyrithiobac(-sodium), Quinchlorac, Quinmerac, Quinoclamine, Quizalofop(-P-ethyl), Quizalofop(-P-tefuryl), Rimsulfuron, Sethoxydim, Simazine, Simetryn, Sulcotrione, Sulfentrazone, Sulfometuron(-methyl), Sulfosate, Sulfosulfuron, Tebutam, Tebuthiuron, Terbuthylazine, Terbutryn, Thenylchlor, Thiafluamide, Thiazopyr, Thidiazimin, Thifensulfuron(-methyl), Thiobencarb, Tiocarbazil, Tralkoxydim, Triallate, Triasulfuron, Tribenuron(-methyl), Triclopyr, Tridiphane, Trifluralin und Triflusulfuron.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Beispiel 1

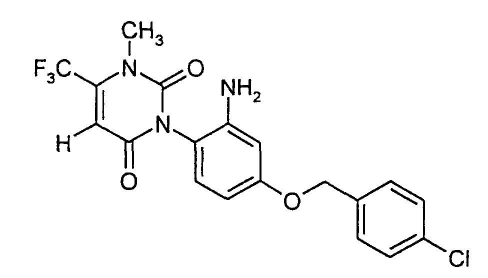

### (Verfahren (c))

12,0 g (26,3 mMol) 1-[4-(4-Chlor-phenylmethoxy)-2-nitro-phenyl]-3-methyl-4-trifluormethyl-3,6-dihydro-2,6-dioxo-1(2H)-pyrimidin werden in 120 ml Essigsäure und 20 ml Wasser vorgelegt und bei 50°C unter Rühren mit 7,53 g (134 mMol) Eisen (Pulver) portionsweise versetzt. Die Reaktionsmischung wird dann noch zwei Stunden bei Raumtemperatur (ca. 20°C) gerührt und anschließend im Wasserstrahlvakuum eingeengt. Der Rückstand wird mit Wasser/Essigsäureethylester geschüttelt, die organische Phase mit Natriumsulfat getrocknet und filtriert. Das Filtrat wird im Wasserstrahlvakuum eingeengt, der Rückstand mit Diethylether/Petrolether digeriert und das kristallin angefallene Produkt durch Absaugen isoliert.

Man erhält 10,5 g (94% der Theorie) 1-[2-Amino-4-(4-chlor-phenylmethoxy)-phenyl]-3-methyl-4-trifluormethyl-3,6-dihydro-2,6-dioxo-1(2H)-pyrimidin vom Schmelzpunkt 166°C.

### Beispiel 2

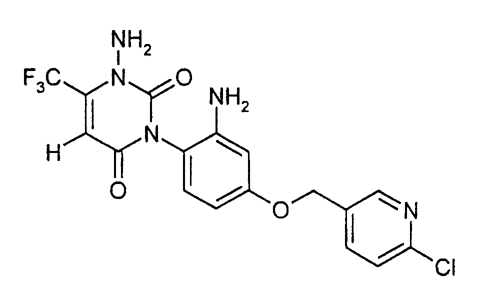

### (Verfahren (c))

4,0 g (8,74 mMol) 1-[4-(2-Chlor-pyridin-5-yl-methoxy)-2-nitro-phenyl]-3-methyl-4-trifluormethyl-3,6-dihydro-2,6-dioxo-1(2H)-pyrimidin werden in 30 ml Essigsäure und 10 ml Wasser vorgelegt und bei 50°C bis 80°C unter Rühren mit 2,67 g (48 mMol) Eisen (Pulver) portionsweise versetzt. Die Reaktionsmischung wird dann noch zwei Stunden bei Raumtemperatur (ca. 20°C) gerührt und anschließend im Wasserstrahlvakuum eingeengt. Der Rückstand wird mit Wasser/Essigsäureethylester geschüttelt, die organische Phase mit Wasser gewaschen, mit Natriumsulfat getrocknet und filtriert. Das Filtrat wird im Wasserstrahlvakuum eingeengt, der Rückstand mit Diethylether/Petrolether digeriert und das kristallin angefallene Produkt durch Absaugen isoliert.

Man erhält 3,65 g (98% der Theorie) 1-[2-Amino-4-(2-chlor-pyridin-5-yl-methoxy)-phenyl]-3-methyl-4-trifluormethyl-3,6-dihydro-2,6-dioxo-1(2H)-pyrimidin vom Schmelzpunkt 109°C.

### Beispiel 3

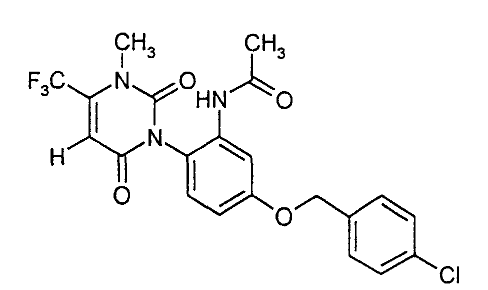

### (Verfahren (d))

Eine Mischung aus 1,0 g (2,35 mMol) 1-[2-Amino-4-(4-chlor-phenylmethoxy)-phenyl]-3-methyl-4-trifluormethyl-3,6-dihydro-2,6-dioxo-1(2H)-pyrimidin, 0,24 g (3,1 mMol) Acetylchlorid, 0,30 g Triethylamin und20 ml Acetonitril wird zwei Stunden unter Rückfluß erhitzt und anschließend im Wasserstrahlvakuum eingeengt. Der Rückstand wird mit 2N-Salzsäure/Essigsäureethylester/Diethylether verrührt und das kristallin angefallene Produkt durch Absaugen isoliert.

Man erhält 0,60 g (55% der Theorie) 1-[2-Acetylamino-4-(4-chlor-phenylmethoxy)-phenyl]-3-methyl-4-trifluormethyl-3,6-dihydro-2,6-dioxo-1(2H)-pyrimidin vom Schmelzpunkt 230°C.

### Beispiel 4

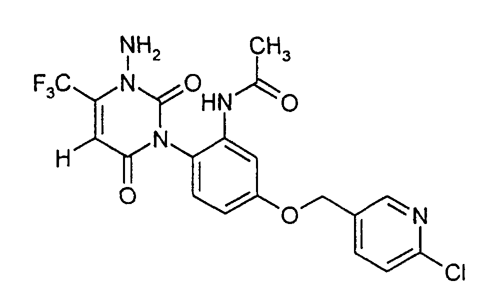

### (Verfahren (d))

Eine Mischung aus 1,0 g (2,34 mMol) 1-[2-Amino-4-(2-chlor-pyridin-5-yl-methoxy)-phenyl]-3-methyl-4-trifluormethyl-3,6-dihydro-2,6-dioxo-1(2H)-pyrimidin, 0,23 g (3,0 mMol) Acetylchlorid, 3,0 g Triethylamino und 10 ml Acetonitril wird 15 Minuten bei 60°C gerührt und anschließend im Wasserstrahlvakuum eingeengt. Der Rückstand wird mit 2N-Salzsäure/Essigsäureethylester/Diethylether verrührt und das kristallin angefallene Produkt durch Absaugen isoliert.

Man erhält 0,50 g (46% der Theorie) 1-[2-Acetylamino-4-(2-chlor-pyridin-5-ylmethoxy)-phenyl]-3-methyl-4-trifluormethyl-3,6-dihydro-2,6-dioxo-1(2H)-pyrimidin vom Schmelzpunkt 228°C.

Analog zu den Herstellungsbeispielen 1 bis 4 sowie entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

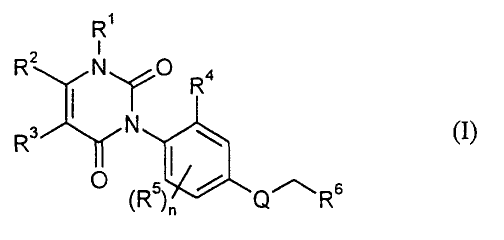

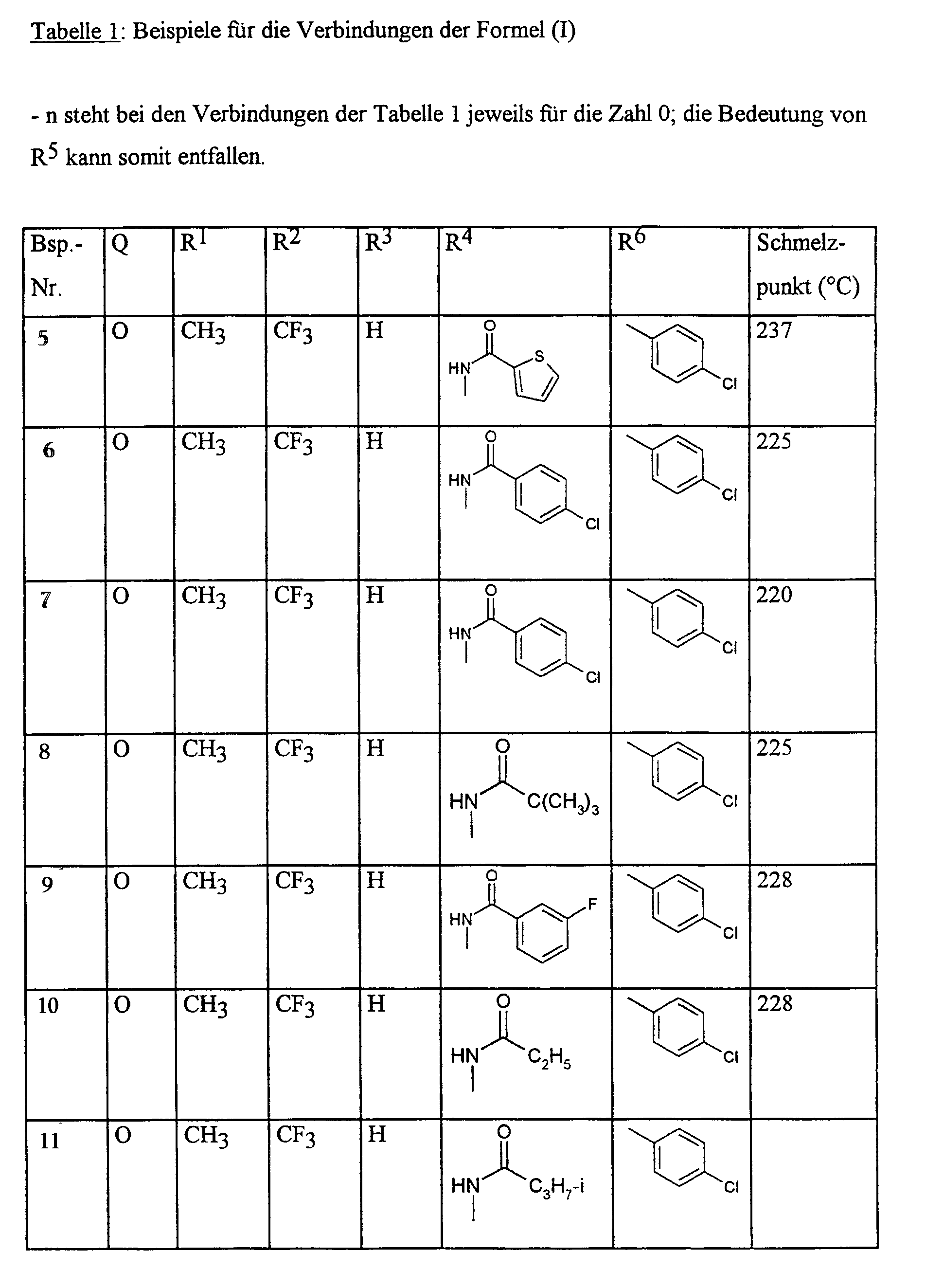

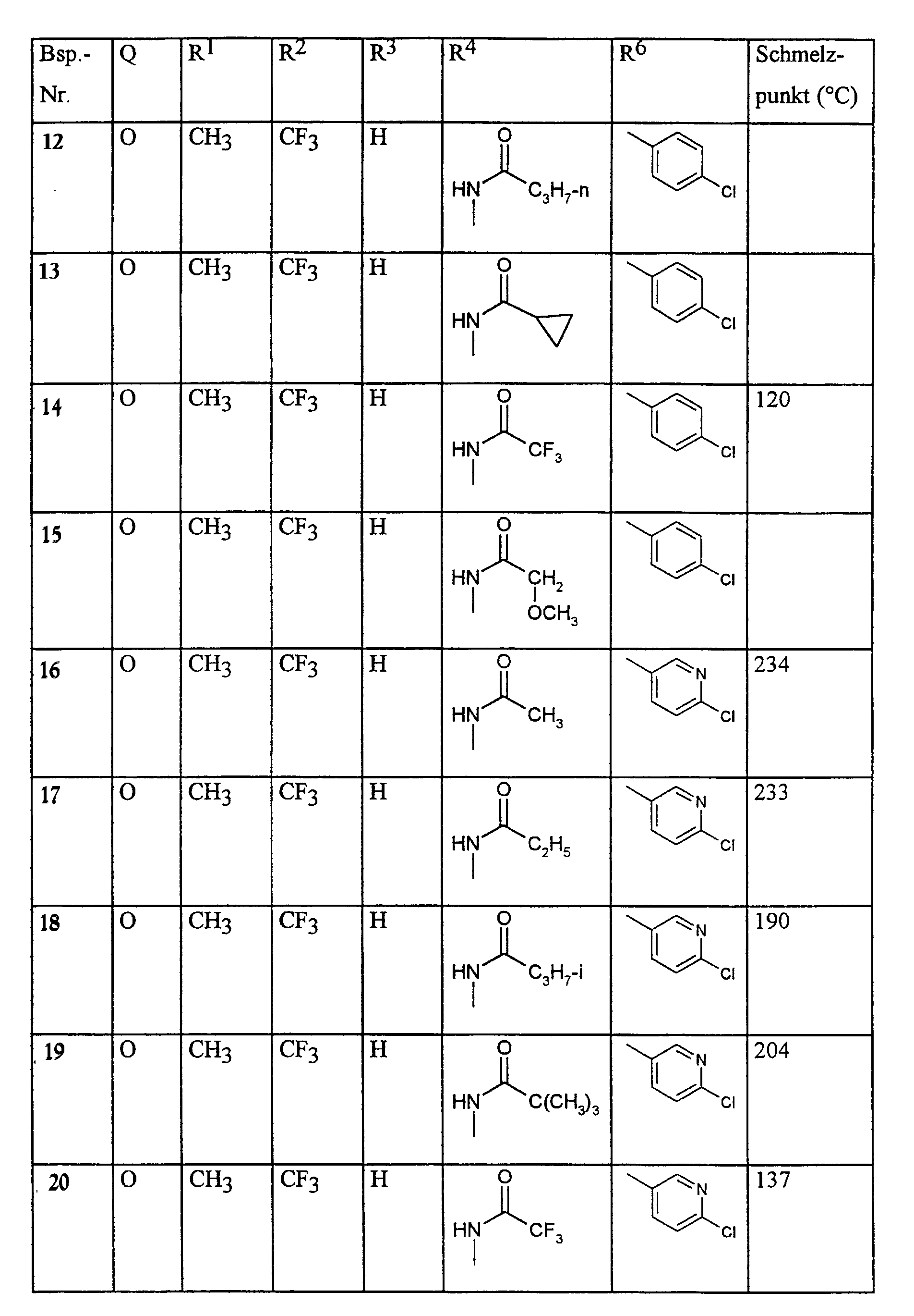

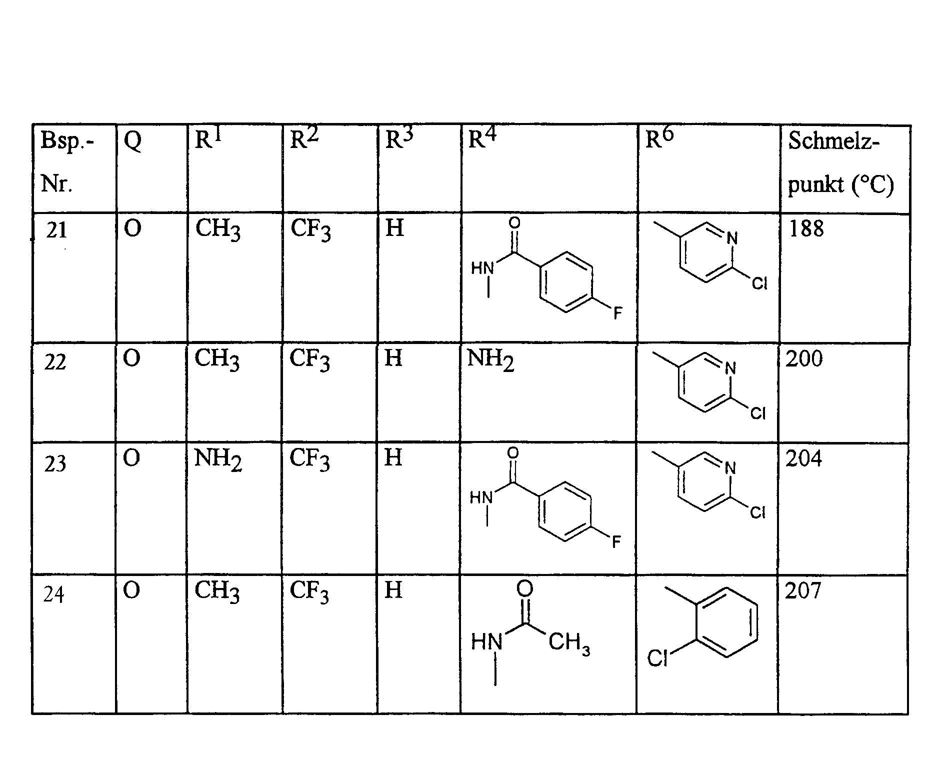

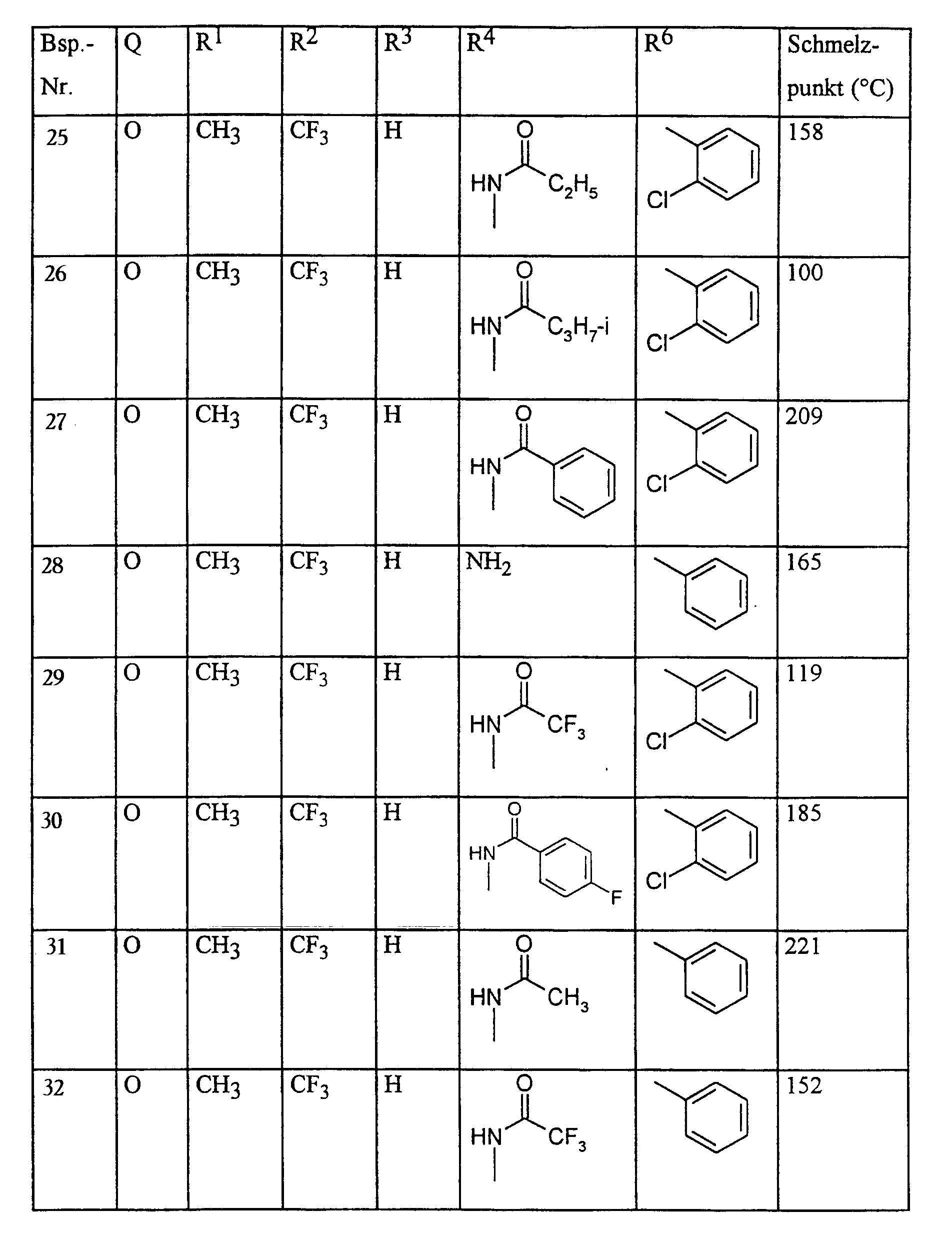

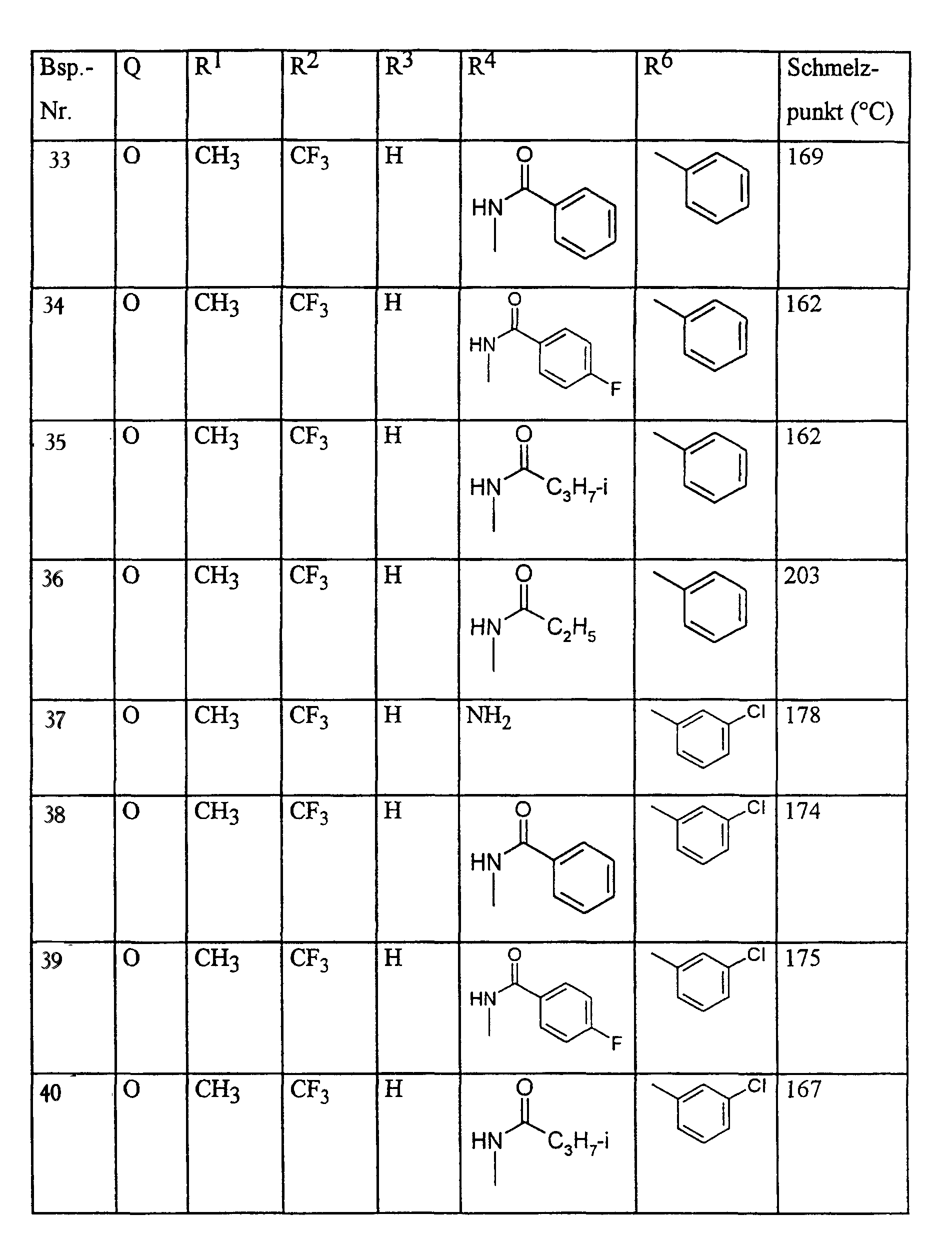

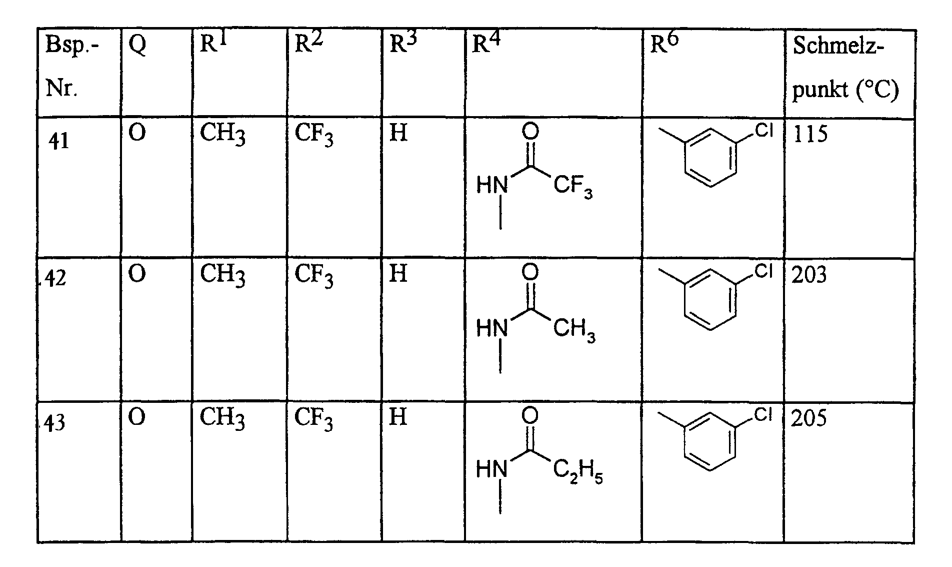

### Ausgangsstoffe der Formel (III):

### Beispiel (III-1)

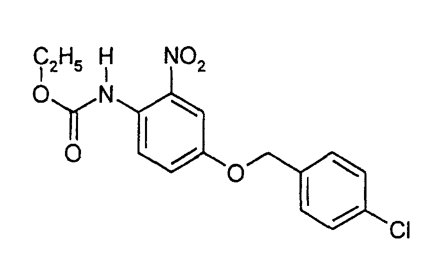

18,1 g (65 mMol) 1-Amino-4-(4-chlor-phenylmethoxy)-2-nitro-benzol werden in 100 ml Aceton vorgelegt und bei 40°C unter Rühren mit 16,3 g ( mMol) Chlorameisensäure-trichlormethylester ("Diphosgen") (83 mMol) tropfenweise versetzt. Die Reaktionsmischung wird 20 Minuten bei 40°C gerührt, dann bei Raumtemperatur (ca. 20°C) zu 200 ml Ethanol tropfenweise gegeben, weitere 15 Minuten bei Raumtemperatur gerührt und anschließend im Wasserstrahlvakuum eingeengt. Der Rückstand wird mit Wasser/Diethylether/Petrolether verrührt und das kristalline Produkt durch Absaugen isoliert.

Man erhält 19,3 g (85% der Theorie) N-[4-(4-Chlor-phenylmethoxy)-2-nitro-phenyl]-O-ethyl-carbamat vom Schmelzpunkt 122°C.

Analog Beispiel (III-1) können beispielsweise auch die in der nachstehenden Tabelle 2 aufgeführten Verbindungen der Formel (III) hergestellt werden.

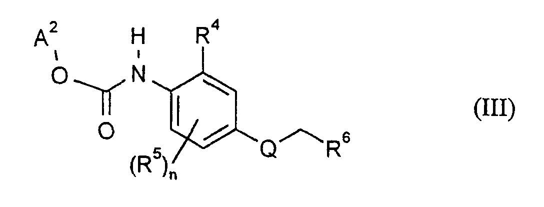

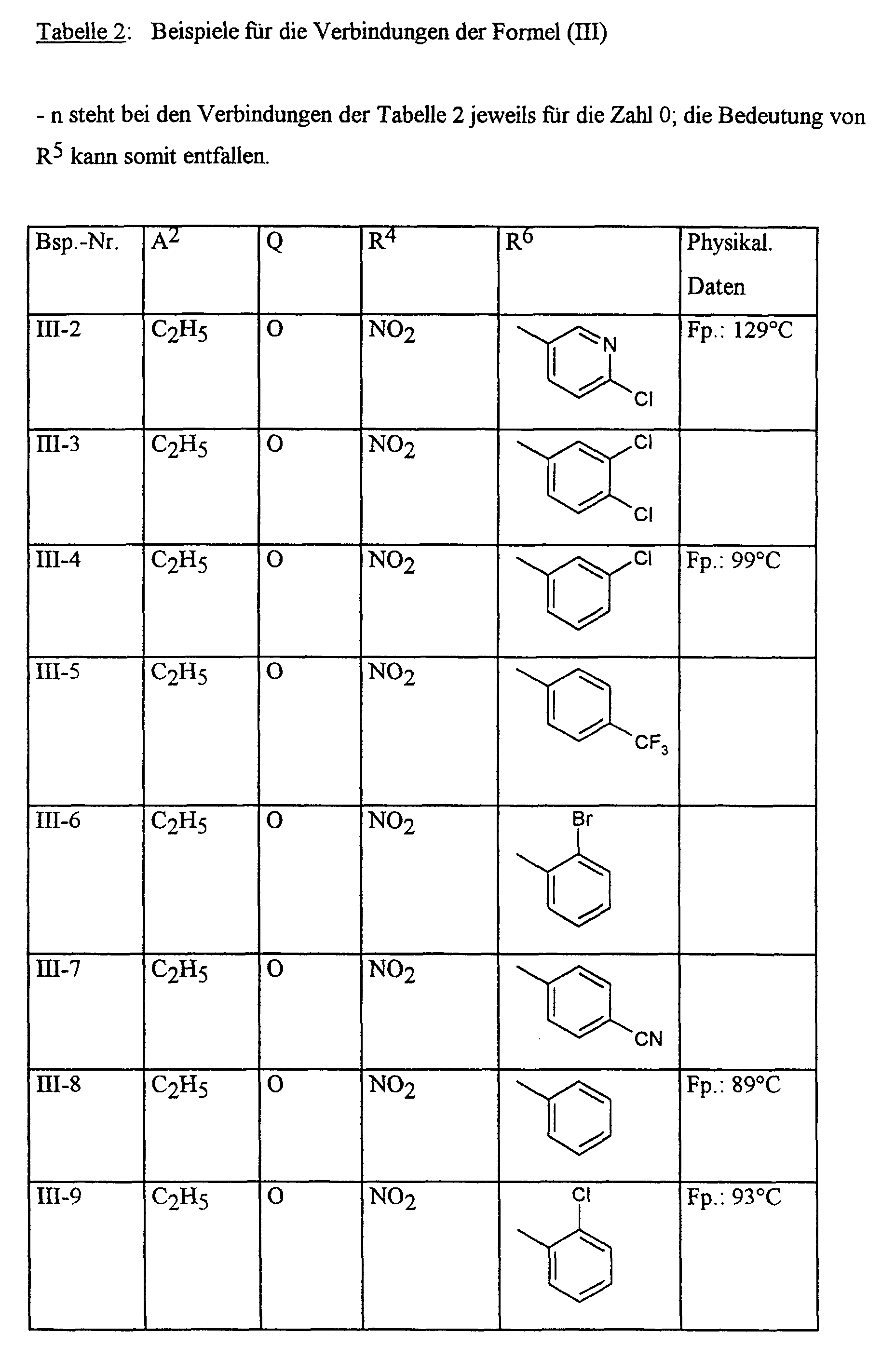

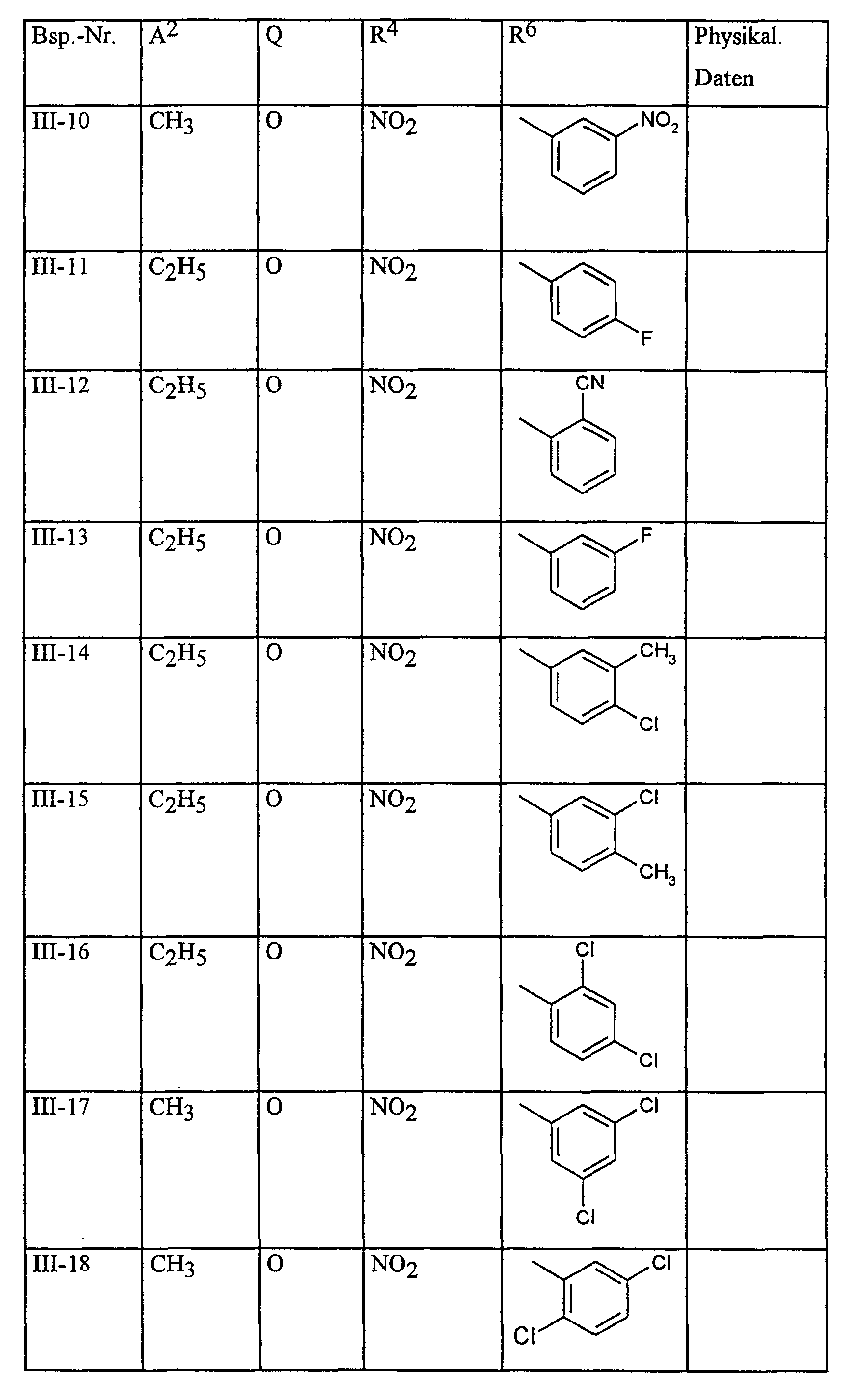

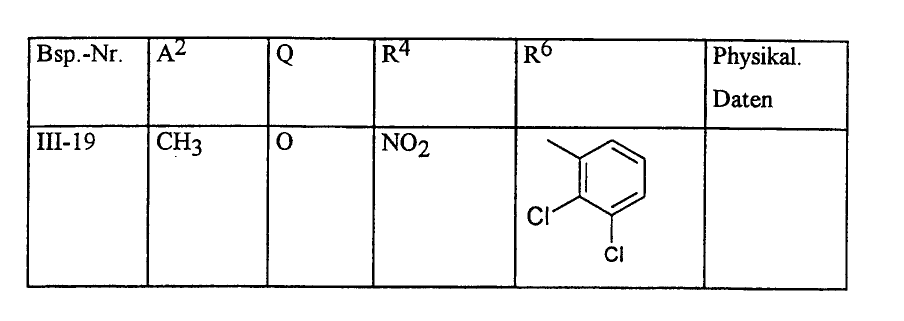

### Ausgangsstoffe der Formel (VI):

### Beispiel (VI-1)

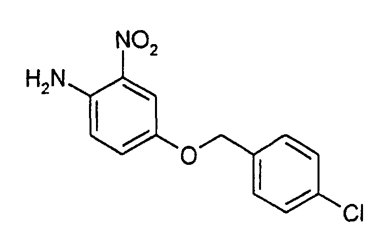

12,0 g (78 mMol) 4-Hydroxy-2-nitro-anilin werden in 50 ml N-Methyl-pyrrolidon vorgelegt und unter Rühren werden nacheinander 2,5 g Natriumhydrid und 12,0 g (78 mMol) 4-Chlor-benzylchlorid dazu gegeben. Die Reaktionsmischung wird 20 Stunden bei Raumtemperatur (ca. 20°C) gerührt, anschließend mit 50 ml Diethylether versetzt, dann auf Eiswasser gegeben und mit Natriumdihydrogenphosphat auf pH=6 eingestellt. Das kristallin angefallene Produkt wird durch Absaugen isoliert.

Man erhält 18,1 g (83,5% der Theorie) 1-Amino-4-(4-chlor-phenylmethoxy)-2-nitrobenzol.

Analog Beispiel (VI-1) können beispielsweise auch die in der nachstehenden Tabelle 3 aufgeführten Verbindungen der Formel (VI) hergestellt werden.

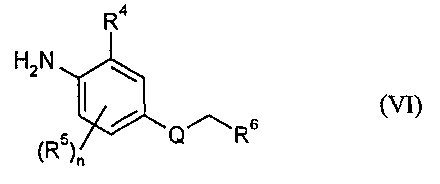

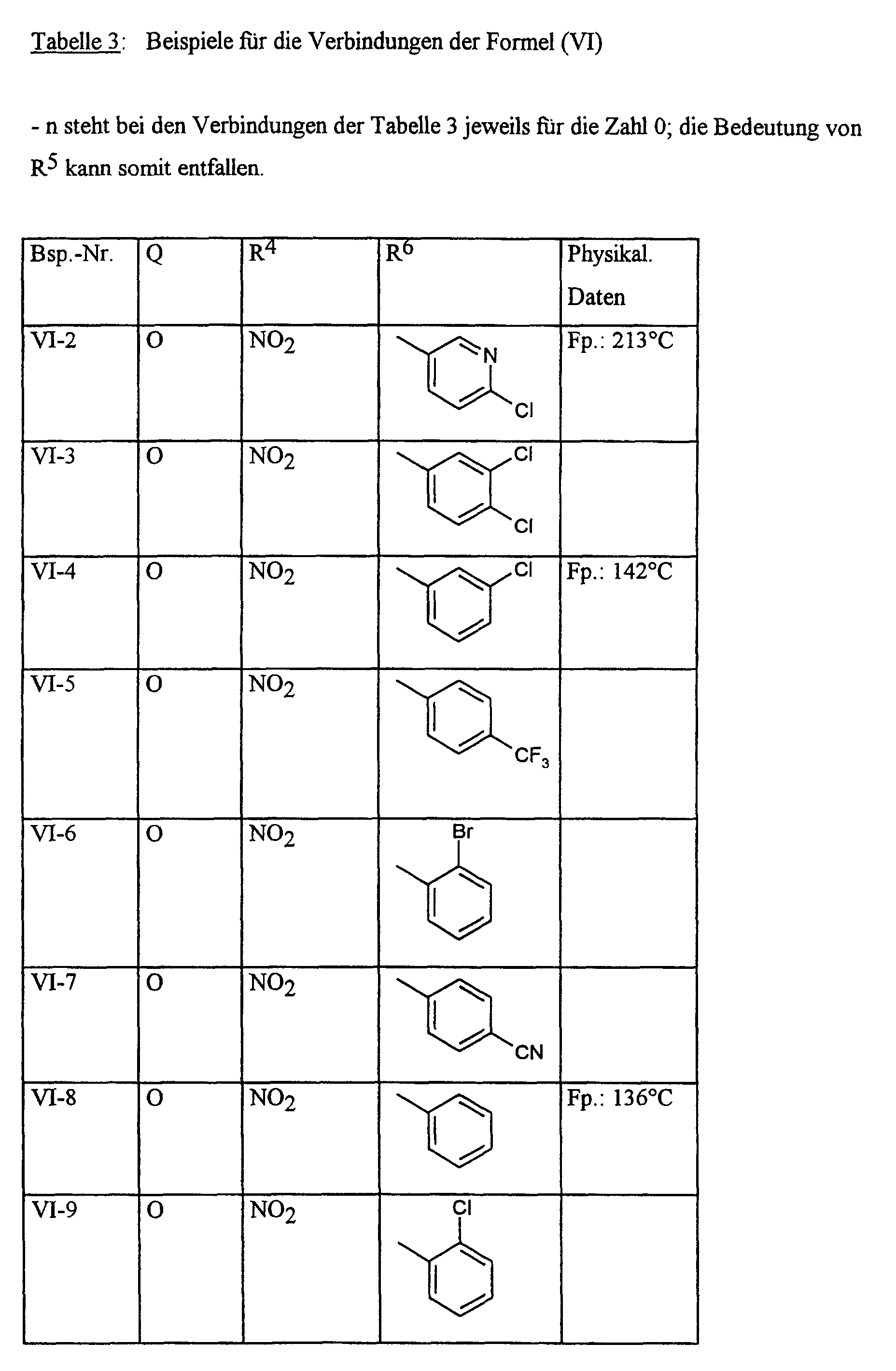

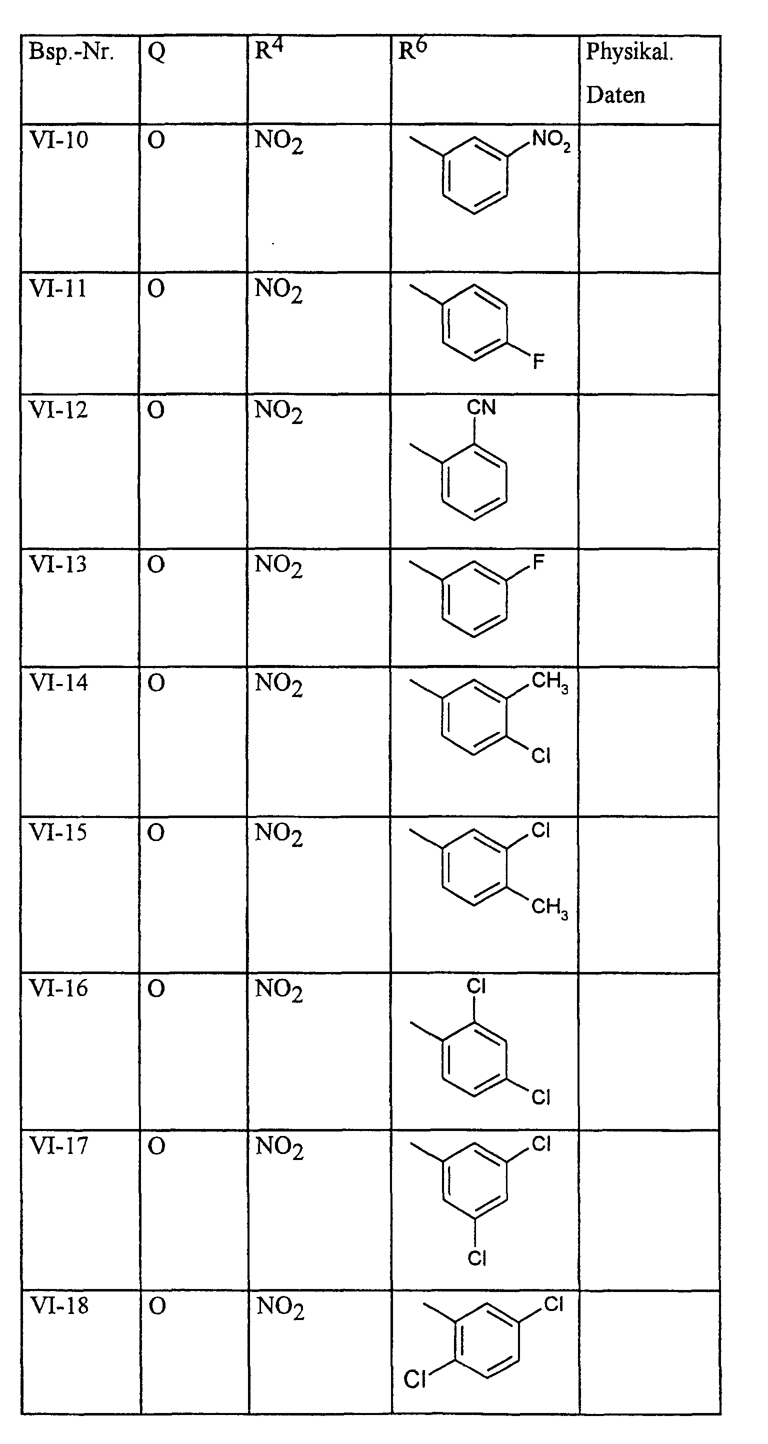

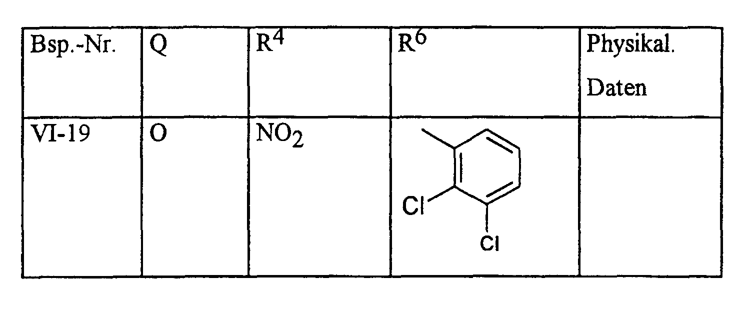

### Anwendungsbeispiele:

### Beispiel A

Pre-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalem Boden ausgesät. Nach ca. 24 Stunden wird der Boden mit der Wirkstoffzubereitung so bespritzt, daß die jeweils gewünschten Wirkstoffmengen pro Flächeneinheit ausgebracht werden. Die Konzentration der Spritzbrühe wird so gewählt, daß in 1 000 l Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % = | keine Wirkung (wie unbehandelte Kontrolle) |
| 100 % = | totale Vernichtung |

In diesem Test zeigen beispielsweise die Verbindungen gemäß Herstellungsbeispiel 3, 5 und 7 bei teilweise guter Verträglichkeit gegenüber Kulturpflanzen, wie z.B. Mais, starke Wirkung gegen Unkräuter.

### Beispiel B

Post-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % = | keine Wirkung (wie unbehandelte Kontrolle) |
| 100 % = | totale Vernichtung |

In diesem Test zeigen beispielsweise die Verbindungen gemäß Herstellungsbeispiel 1, 3, 5, 6 7 und 9 bei teilweise guter Verträglichkeit gegenüber Kulturpflanzen, wie z.B. Mais und Weizen, starke Wirkung gegen Unkräuter.

## Patentansprüche

1. Substituierte Phenyluracile der allgemeinen Formel (I) 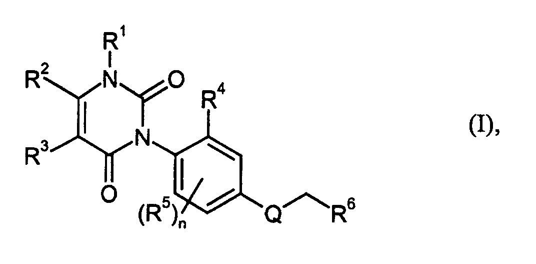 in welcher
n für die Zahlen 0, 1, 2 oder 3 steht,
Q für O, S, SO, SO₂, NH oder N(C₁-C₄-Alkyl) steht,
R¹ für Wasserstoff, Amino oder gegebenenfalls durch Cyano, Halogen oder C₁-C₄-Alkoxy substituiertes Alkyl mit 1 bis 4 Kohlenstoffatomen steht,
R² für Carboxy, Cyano, Carbamoyl, Thiocarbamoyl oder für jeweils gegebenenfalls durch Cyano, Halogen oder C₁-C₄-Alkoxy substituiertes Alkyl oder Alkoxycarbonyl mit jeweils 1 bis 4 Kohlenstoffatomen steht,
R³ für Wasserstoff, Halogen oder gegebenenfalls durch Halogen substituiertes Alkyl mit 1 bis 4 Kohlenstoffatomen steht,
R⁴ für Amino oder für eine der nachstehenden Gruppierungen -NH-CO-R⁷ oder -N(CO-R⁷)₂ steht,
R⁵ für Nitro, Amino, Hydroxy, Mercapto, Carboxy, Cyano, Carbamoyl, Thiocarbamoyl, Sulfo, Chlorsulfonyl, Aminosulfonyl, Halogen, oder für jeweils gegebenenfalls durch Cyano, Halogen oder C₁-C₄-Alkoxy substituiertes Alkyl, Alkoxy, Alkylthio, Alkylsulfinyl, Alkylsulfonyl, Alkylamino, Alkylcarbonyl, Alkoxycarbonyl, Alkylaminocarbonyl, Alkylcarbonylamino, Alkoxycarbonylamino, Alkylsulfonylamino oder Bis-alkylsulfonyl-amino mit jeweils 1 bis 6 Kohlenstoffatomen in den Alkylgruppen steht,
R⁶ für jeweils gegebenenfalls durch Nitro, Cyano, Carboxy, Carbamoyl, Thiocarbamoyl, Sulfo, Chlorsulfonyl, Aminosulfonyl, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl oder C₁-C₄-Alkoxy-carbonyl substituiertes Phenyl, Naphthyl oder Heterocyclyl aus der Reihe Furyl, Tetrahydrofuryl, Benzofuryl, Dihydrobenzofuryl, Dioxolanyl, Dioxanyl, Benzodioxanyl, Pyrrolyl, Pyrazolyl, Imidazolyl, Benzimidazolyl, Triazolyl, Oxazolyl, Isoxazolyl, Benzoxazolyl, Thiazolyl, Isothiazolyl, Benzthiazolyl, Pyridinyl, Chinolinyl, Isochinolinyl, Pyrimidinyl, Pyrazinyl, Pyridazinyl, Chinazolinyl, Chinoxalinyl steht, und
R⁷ für Wasserstoff, für jeweils gegebenenfalls durch Cyano, Halogen oder C₁-C₄-Alkoxy substituiertes Alkyl, Alkoxy, Alkylthio, Alkylamino oder Dialkylamino mit jeweils 1 bis 6 Kohlenstoffatomen in den Alkylgruppen, für jeweils gegebenenfalls durch Cyano oder Halogen substituiertes Alkenyl oder Alkinyl mit jeweils 2 bis 6 Kohlenstoffatomen, für jeweils gegebenenfalls durch Cyano, Halogen oder C₁-C₄-Alkyl substituiertes Cycloalkyl oder Cycloalkylalkyl mit jeweils 3 bis 6 Kohlenstoffatomen in den Cycloalkylgruppen und gegebenenfalls 1 bis 4 Kohlenstoffatomen im Alkylteil, oder für jeweils gegebenenfalls durch Nitro, Cyano, Carboxy, Carbamoyl, Thiocarbamoyl, Sulfo, Chlorsulfonyl, Aminosulfonyl, Halogen, C₁-C₄Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl oder C₁-C₄-Alkoxy-carbonyl substituiertes Phenyl, steht.

2. Substituierte Phenyluracile der allgemeinen Formel (I) nach Anspruch 1, **dadurch gekennzeichnet, dass**
n für die Zahlen 0, 1 oder 2 steht,
Q für O, S, SO, SO₂, NH oder N(CH₃) steht,
R¹ für Wasserstoff, Amino oder jeweils gegebenenfalls durch Cyano, Fluor, Chlor, Methoxy oder Ethoxy substituiertes Methyl, Ethyl, n- oder i-Propyl steht,
R² für Carboxy, Cyano, Carbamoyl, Thiocarbamoyl oder für jeweils gegebenenfalls durch Cyano, Fluor, Chlor, Methoxy oder Ethoxy substituiertes Methyl, Ethyl, n- oder i-Propyl, Methoxycarbonyl, Ethoxycarbonyl, n- oder i-Propoxycarbonyl steht,
R³ für Wasserstoff, Fluor, Chlor, Brom oder für jeweils gegebenenfalls durch Fluor und/oder Chlor substituiertes Methyl, Ethyl, n- oder i-Propyl steht,
R⁴ für Amino oder für eine der nachstehenden Gruppierungen -NH-CO-R⁷ oder -N(CO-R⁷)₂ steht,
R⁵ für Nitro, Amino, Hydroxy, Mercapto, Carboxy, Cyano, Carbamoyl, Thiocarbamoyl, Sulfo, Chlorsulfonyl, Aminosulfonyl, Fluor, Chlor, Brom, oder für jeweils gegebenenfalls durch Cyano, Fluor, Chlor, Methoxy oder Ethoxy substituiertes Methyl, Ethyl, n- oder i-Propyl, Methoxy, Ethoxy, n- oder i-Propoxy, Methylthio, Ethylthio, n- oder i-Propylthio, Methylsulfinyl, Ethylsulfinyl, Methylsulfonyl, Ethylsulfonyl, Methylamino, Ethylamino, n- oder i-Propylamino, Acetyl, Propionyl, Methoxycarbonyl, Ethoxycarbonyl, Methylaminocarbonyl, Ethylaminocarbonyl, Acetylamino, Propionylamino, Methoxycarbonylamino, Ethoxycarbonylamino, Methylsulfonylamino, Ethylsulfonylamino, Bis-methylsulfonyl-amino oder Bis-ethylsulfonylamino steht,
R⁶ für jeweils gegebenenfalls durch Nitro, Cyano, Carboxy, Carbamoyl, Thiocarbamoyl, Sulfo, Chlorsulfonyl, Aminosulfonyl, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl oder C₁-C₄-Alkoxy-carbonyl substituiertes Phenyl, Naphthyl oder Heterocyclyl aus der Reihe Furyl, Tetrahydrofuryl, Benzofuryl, Dihydrobenzofuryl, Dioxolanyl, Dioxanyl, Benzodioxanyl, Pyrrolyl, Pyrazolyl, Imidazolyl, Benzimidazolyl, Triazolyl, Oxazolyl, Isoxazolyl, Benzoxazolyl, Thiazolyl, Isothiazolyl, Benzthiazolyl, Pyridinyl, Chinolinyl, Isochinolinyl, Pyrimidinyl, Pyrazinyl, Pyridazinyl, Chinazolinyl, Chinoxalinyl steht, und
R⁷ für Wasserstoff, für jeweils gegebenenfalls durch Cyano, Halogen oder C₁-C₄-Alkoxy substituiertes Alkyl, Alkoxy, Alkylthio, Alkylamino oder Dialkylamino mit jeweils 1 bis 6 Kohlenstoffatomen in den Alkylgruppen, für jeweils gegebenenfalls durch Cyano oder Halogen substituiertes Alkenyl oder Alkinyl mit jeweils 2 bis 6 Kohlenstoffatomen, für jeweils gegebenenfalls durch Cyano, Halogen oder C₁-C₄-Alkyl substituiertes Cycloalkyl oder Cycloalkylalkyl mit jeweils 3 bis 6 Kohlenstoffatomen in den Cycloalkylgruppen und gegebenenfalls 1 bis 4 Kohlenstoffatomen im Alkylteil, oder für jeweils gegebenenfalls durch Nitro, Cyano, Carboxy, Carbamoyl, Thiocarbamoyl, Sulfo, Chlorsulfonyl, Aminosulfonyl, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl oder C₁-C₄-Alkoxy-carbonyl substituiertes Phenyl, steht.

3. Substituierte Phenyluracile der allgemeinen Formel (I) nach Anspruch 1, **dadurch gekennzeichnet, dass**
R¹ für Wasserstoff steht.

4. Substituierte Phenyluracile der allgemeinen Formel (I) nach Anspruch 1, **dadurch gekennzeichnet, dass**
R⁴ für Amino steht.

5. Verfahren zur Herstellung von substituierten Phenyluracilen der allgemeinen Formel (I) nach einem der Ansprüche 1 bis 4, **gekennzeichnet durch** Umsetzen von
(a) Aminoalkensäureestern der allgemeinen Formel (II) 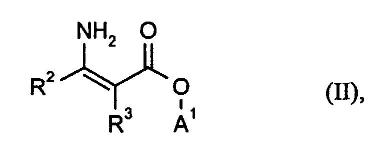 in welcher
R² und R³ die in Anspruch 1 oder 2 angegebenen Bedeutungen haben und
A¹ für C₁-C₄-Alkyl, Phenyl oder Benzyl steht,
mit substituierten Phenylurethanen (Phenylcarbamaten) der allgemeinen Formel (III) 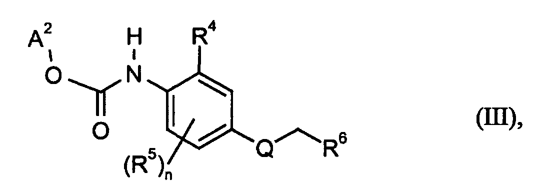 in welcher
n, Q, R⁴, R⁵ und R⁶ die in Anspruch 1 oder 2 angegebenen Bedeutungen haben und
A² für C₁-C₄-Alkyl, Phenyl oder Benzyl, steht,
gegebenenfalls in Gegenwart eines Reaktionshilfsmittels und gegebenenfalls in Gegenwart eines Verdünnungsmittels oder **durch** Umsetzen von
(b) substituierten Phenyluracilen der allgemeinen Formel (Ia), 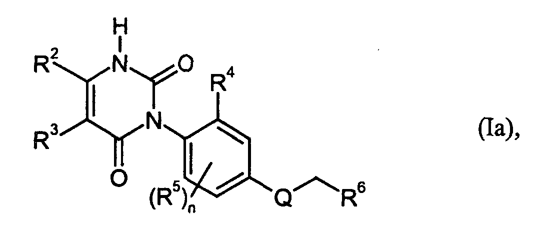 in welcher
n, Q, R², R³, R⁴, R⁵ und R⁶ die in Anspruch 1 oder 2 angegebenen Bedeutungen haben,
mit 1-Aminooxy-2,4-dinitro-benzol oder mit Alkylierungsmitteln der allgemeinen Formel (IV)
X¹-A³ (IV)
in welcher
A³ für gegebenenfalls **durch** Cyano, Halogen oder C₁-C₄-Alkoxy substituiertes Alkyl mit 1 bis 4 Kohlenstoffatomen steht und
X¹ für Chlor, Brom, Iod oder die Gruppierung Methylsulfonyloxy oder Ethylsulfonyloxy steht,
gegebenenfalls in Gegenwart eines Reaktionshilfsmittels und gegebenenfalls in Gegenwart eines Verdünnungsmittels
oder **durch** Umsetzen von
(c) substituierten Phenyluracilen der allgemeinen Formel (Ib), 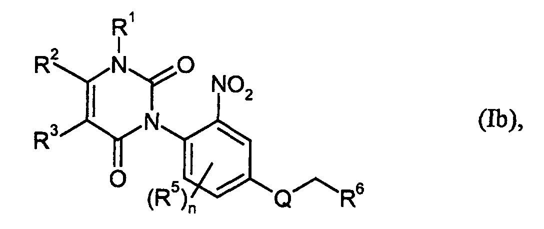 in welcher
n, Q, R¹, R², R³, R⁵ und R⁶ die in Anspruch 1 oder 2 angegebenen Bedeutungen haben,
mit Hydrierungsmitteln gegebenenfalls in Gegenwart von Reaktionshilfsmitteln und gegebenenfalls in Gegenwart von Verdünnungsmitteln
oder **durch** Umsetzen von
(d) substituierten Phenyluracilen der allgemeinen Formel (Ic), 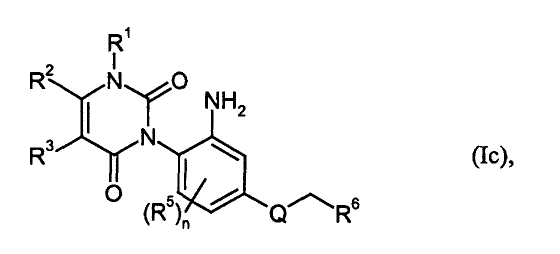 in welcher
n, Q, R¹, R², R³, R⁵ und R⁶ die in Anspruch 1 oder 2 angegebenen Bedeutungen haben,
mit Acylierungsmitteln der allgemeinen Formel (V)
X²-CO-R⁷ (V)
in welcher
R⁷ die in Anspruch 1 oder 2 angegebene Bedeutung hat und
X² für Fluor, Chlor oder Brom steht,
gegebenenfalls in Gegenwart von Reaktionshilfsmitteln und gegebenenfalls in Gegenwart von Verdünnungsmitteln.

6. Verwendung von mindestens einem substituierten Phenyluracil nach einem der Ansprüche 1 bis 4 zur Bekämpfung von unerwünschten Pflanzen.

7. Herbizides Mittel, **gekennzeichnet durch** den Gehalt an mindestens einem substituierten Phenyluracil nach einem der Ansprüche 1 bis 4.

## Claims

1. Substituted phenyluracils of the general formula (I) 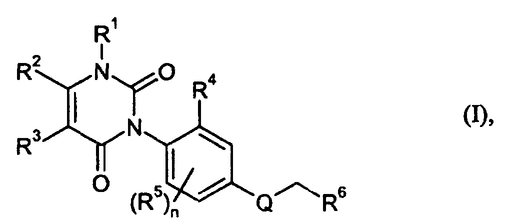 in which
n represents the number 0, 1, 2 or 3,
Q represents O, S, SO, SO₂, NH or N(C₁-C₄-alkyl),
R¹ represents hydrogen, amino or optionally cyano-, halogen- or C₁-C₄-alkoxy-substituted alkyl having 1 to 4 carbon atoms,
R² represents carboxyl, cyano, carbamoyl, thiocarbamoyl or represents in each case optionally cyano-, halogen- or C₁-C₄-alkoxysubstituted alkyl or alkoxycarbonyl having in each case 1 to 4 carbon atoms,
R³ represents hydrogen, halogen or optionally halogen-substituted alkyl having 1 to 4 carbon atoms,
R⁴ represents amino or represents one of the groupings below -NH-CO-R⁷ or -N(CO-R⁷)₂,
R⁵ represents nitro, amino, hydroxyl, mercapto, carboxyl, cyano, carbamoyl, thiocarbamoyl, sulfo, chlorosulfonyl, aminosulfonyl, halogen, or represents in each case optionally cyano-, halogen- or C₁-C₄-alkoxy-substituted alkyl, alkoxy, alkylthio, alkylsulfinyl, alkylsulfonyl, alkylamino, alkylcarbonyl, alkoxycarbonyl, alkylaminocarbonyl, alkylcarbonylamino, alkoxycarbonylamino, alkylsulfonylamino or bis-alkylsulfonyl-amino having in each case 1 to 6 carbon atoms in the alkyl groups,
R⁶ represents in each case optionally nitro-, cyano-, carboxyl-, carbamoyl-, thiocarbamoyl-, sulfo-, chlorosulfonyl-, aminosulfonyl-, halogen-, C₁-C₄-alkyl-, C₁-C₄-halogenoalkyl-, C₁-C₄-alkoxy-, C₁-C₄-halogenoalkoxy-, C₁-C₄-alkylthio-, C₁-C₄-halogenoalkylthio-, C₁-C₄-alkylsulfinyl-, C₁-C₄-halogenoalkylsulfinyl-, C₁-C₄-alkylsulfonyl-, C₁-C₄-halogenoalkylsulfonyl- or C₁-C₄-alkoxy-carbonyl-substituted phenyl, naphthyl or heterocyclyl from the group consisting of furyl, tetrahydrofuryl, benzofuryl, dihydrobenzofuryl, dioxolanyl, dioxanyl, benzodioxanyl, pyrrolyl, pyrazolyl, imidazolyl, benzimidazolyl, triazolyl, oxazolyl, isoxazolyl, benzoxazolyl, thiazolyl, isothiazolyl, benzothiazolyl, pyridinyl, quinolinyl, isoquinolinyl, pyrimidinyl, pyrazinyl, pyridazinyl, quinazolinyl, quinoxalinyl, and
R⁷ represents hydrogen, represents in each case optionally cyano-, halogen- or C₁-C₄-alkoxy-substituted alkyl, alkoxy, alkylthio, alkylamino or dialkylamino having in each case 1 to 6 carbon atoms in the alkyl groups, represents in each case optionally cyano- or halogen-substituted alkenyl or alkinyl having in each case 2 to 6 carbon atoms, represents in each case optionally cyano-, halogen- or C₁-C₄-alkyl-substituted cycloalkyl or cycloalkylalkyl having in each case 3 to 6 carbon atoms in the cycloalkyl groups and optionally 1 to 4 carbon atoms in the alkyl moiety, or represents in each case optionally nitro-, cyano-, carboxyl-, carbamoyl-, thiocarbamoyl-, sulfo-, chlorosulfonyl-, aminosulfonyl-, halogen-, C₁-C₄-alkyl-, C₁-C₄-halogenoalkyl-, C₁-C₄-alkoxy-, C₁-C₄-halogenoalkoxy-, C₁-C₄-alkylthio-, C₁-C₄-halogenoalkylthio-, C₁-C₄-alkylsulfinyl-, C₁-C₄-halogenoalkylsulfinyl-, C₁-C₄-alkylsulfonyl-, C₁-C₄-halogenoalkylsulfonyl- or C₁-C₄-alkoxycarbonyl-substituted phenyl.

2. Substituted phenyluracils of the general formula (I) according to Claim 1, **characterized in that**
n represents the number 0, 1 or 2,
Q represents O, S, SO, SO₂, NH or N(CH₃),
R¹ represents hydrogen, amino or in each case optionally cyano-, fluorine-, chlorine-, methoxy- or ethoxy-substituted methyl, ethyl, n- or i-propyl,
R² represents carboxyl, cyano, carbamoyl, thiocarbamoyl or represents in each case optionally cyano-, fluorine-, chlorine-, methoxy- or ethoxy-substituted methyl, ethyl, n- or i-propyl, methoxycarbonyl, ethoxycarbonyl, n- or i-propoxycarbonyl,
R³ represents hydrogen, fluorine, chlorine, bromine or represents in each case optionally fluorine- and/or chlorine-substituted methyl, ethyl, n- or i-propyl,
R⁴ represents amino or represents one of the groupings below -NH-CO-R⁷ or -N(CO-R⁷)₂,
R⁵ represents nitro, amino, hydroxyl, mercapto, carboxyl, cyano, carbamoyl, thiocarbamoyl, sulfo, chlorosulfonyl, aminosulfonyl, fluorine, chlorine, bromine, or represents in each case optionally cyano-, fluorine-, chlorine-, methoxy- or ethoxy-substituted methyl, ethyl, n- or i-propyl, methoxy, ethoxy, n- or i-propoxy, methylthio, ethylthio, n- or i-propylthio, methylsulfinyl, ethylsulfinyl, methylsulfonyl, ethylsulfonyl, methylamino, ethylamino, n- or i-propylamino, acetyl, propionyl, methoxycarbonyl, ethoxycarbonyl, methylaminocarbonyl, ethylaminocarbonyl, acetylamino, propionylamino, methoxycarbonylamino, ethoxycarbonylamino, methylsulfonylamino, ethylsulfonylamino, bis-methylsulfonyl-amino or bis-ethylsulfonyl-amino,
R⁶ represents in each case optionally nitro-, cyano-, carboxyl-, carbamoyl-, thiocarbamoyl-, sulfo-, chlorosulfonyl-, aminosulfonyl-, halogen-, C₁-C₄-alkyl-, C₁-C₄-halogenoalkyl-, C₁-C₄-alkoxy-, C₁-C₄-halogenoalkoxy-, C₁-C₄-alkylthio-, C₁-C₄-halogenoalkylthio-, C₁-C₄-alkylsulfinyl-, C₁-C₄-halogenoalkylsulfinyl-, C₁-C₄-alkylsulfonyl-, C₁-C₄-halogenoalkylsulfonyl- or C₁-C₄-alkoxy-carbonyl-substituted phenyl, naphthyl or heterocyclyl from the group consisting of furyl, tetrahydrofuryl, benzofuryl, dihydrobenzofuryl, dioxolanyl, dioxanyl, benzodioxanyl, pyrrolyl, pyrazolyl, imidazolyl, benzimidazolyl, triazolyl, oxazolyl, isoxazolyl, benzoxazolyl, thiazolyl, isothiazolyl, benzothiazolyl, pyridinyl, quinolinyl, isoquinolinyl, pyrimidinyl, pyrazinyl, pyridazinyl, quinazolinyl, quinoxalinyl, and
R⁷ represents hydrogen, represents in each case optionally cyano-, halogen- or C₁-C₄-alkoxy-substituted alkyl, alkoxy, alkylthio, alkylamino or dialkylamino having in each case 1 to 6 carbon atoms in the alkyl groups, represents in each case optionally cyano- or halogen-substituted alkenyl or alkinyl having in each case 2 to 6 carbon atoms, represents in each case optionally cyano-, halogen- or C₁-C₄-alkyl-substituted cycloalkyl or cycloalkylalkyl having in each case 3 to 6 carbon atoms in the cycloalkyl groups and optionally 1 to 4 carbon atoms in the alkyl moiety, or represents in each case optionally nitro-, cyano-, carboxyl-, carbamoyl-, thiocarbamoyl-, sulfo-, chlorosulfonyl-, aminosulfonyl-, halogen-, C₁-C₄-alkyl-, C₁-C₄-halogenoalkyl-, C₁-C₄-alkoxy-, C₁-C₄-halogenoalkoxy-, C₁-C₄-alkylthio-, C₁-C₄-halogenoalkylthio-, C₁-C₄-alkylsulfinyl-, C₁-C₄-halogenoalkylsulfinyl-, C₁-C₄-alkylsulfonyl-, C₁-C₄-halogenoalkylsulfonyl- or C₁-C₄-alkoxy-carbonyl-substituted phenyl.

3. Substituted phenyluracils of the general formula (I) according to Claim 1, **characterized in that**
R¹ represents hydrogen.

4. Substituted phenyluracils of the general formula (I) according to Claim 1, **characterized in that**
R⁴ represents amino.

5. Process for preparing substituted phenyluracils of the general formula (I) according to any of Claims 1 to 4, **characterized by** reacting
(a) aminoalkenoic esters of the general formula (II) 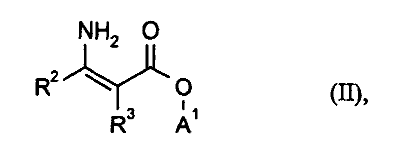 in which
R² and R³ are each as defined in Claim 1 or 2 and
A¹ represents C₁-C₄-alkyl, phenyl or benzyl,
with substituted phenylurethanes (phenylcarbamates) of the general formula (III) 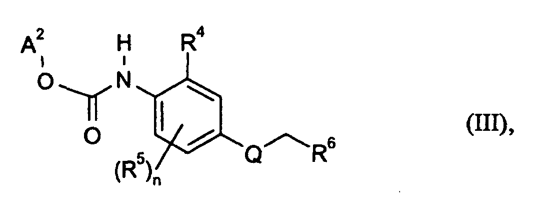 in which
n, Q, R⁴, R⁵ and R⁶ are each as defined in Claim 1 or 2 and
A² represents C₁-C₄-alkyl, phenyl or benzyl,
if appropriate in the presence of a reaction auxiliary and if appropriate in the presence of a diluent,
or by reacting
(b) substituted phenyluracils of the general formula (Ia), 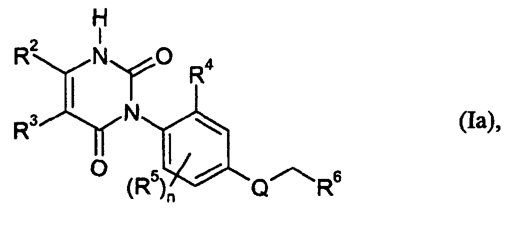 in which
n, Q, R², R³, R⁴, R⁵ and R⁶ are each as defined in Claim 1 or 2,
with 1-aminooxy-2,4-dinitro-benzene or with alkylating agents of the general formula (IV)
X¹-A³ (IV)
in which
A³ represents optionally cyano-, halogen- or C₁-C₄-alkoxy-substituted alkyl having 1 to 4 carbon atoms and
X¹ represents chlorine, bromine, iodine or the grouping methylsulphonyloxy or ethylsulphonyloxy,
if appropriate in the presence of a reaction auxiliary and if appropriate in the presence of a diluent,
or by reacting
(c) substituted phenyluracils of the general formula (Ib), 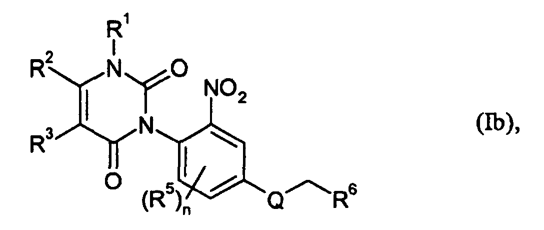 in which
n, Q, R¹, R², R³, R⁵ and R⁶ are each as defined in Claim 1 or 2,
with hydrogenating agents, if appropriate in the presence of reaction auxiliaries and if appropriate in the presence of diluents,
or by reacting
(d) substituted phenyluracils of the general formula (Ic), 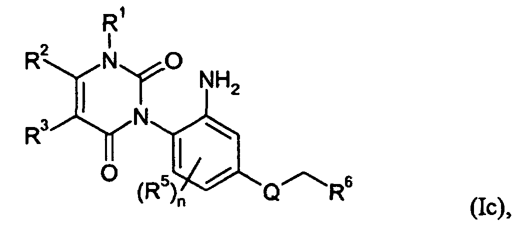 in which
n, Q, R¹, R², R³, R⁵ and R⁶ are each as defined in Claim 1 or 2,
with acylating agents of the general formula (V)
X²-CO-R⁷ (v)
in which
R⁷ is as defined in Claim 1 or 2 and
X² represents fluorine, chlorine or bromine,
if appropriate in the presence of reaction auxiliaries and if appropriate in the presence of diluents.

6. Use of at least one substituted phenyluracil according to any of Claims 1 to 4 for controlling undesirable plants.

7. Herbicidal composition, **characterized in that** it comprises at least one substituted phenyluracil according to any of Claims 1 to 4.

## Revendications

1. Phényluraciles substitués de formule générale (I) 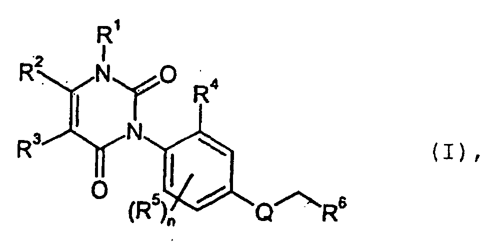 dans laquelle
n représente les nombres 0, 1, 2 ou 3,
Q représente O, S, SO, SO₂, NH ou un groupe N(alkyle en C₁ à C₄),
R¹ représente l'hydrogène, un groupe amino ou un reste alkyle ayant 1 à 4 atomes de carbone éventuellement substitué par un radical cyano, halogéno ou alkoxy en C₁ à C₄,
R² est un groupe carboxy, cyano, carbamoyle, thiocarbamoyle ou un reste alkyle ou alkoxycarbonyle ayant chacun 1 à 4 atomes de carbone et portant chacun un substituant cyano, halogéno ou alkoxy en C₁ à C₄,
R³ représente l'hydrogène, un halogène ou un reste alkyle ayant 1 à 4 atomes de carbone substitué éventuellement par un halogène,
R⁴ est un groupe amino ou l'un des groupements suivants -NH-CO-R⁷ ou -N(CO-R⁷)₂,
R⁵ est un groupe nitro, amino, hydroxy, mercapto, carboxy, cyano, carbamoyle, thiocarbamoyle, sulfo, chlorosulfonyle, aminosulfonyle, un halogène ou un reste alkyle, alkoxy, alkylthio, alkylsulfinyle, alkylsulfonyle, alkylamino, alkylcarbonyle, alkoxycarbonyle, alkylaminocarbonyle, alkylcarbonylamino, alkoxycarbonylamino, alkylsulfonylamino ou bis-alkylsulfonylamino ayant chacun 1 à 6 atomes de carbone dans les groupes alkyle et chacun portant éventuellement un substituant cyano, halogéno ou alkoxy en C₁ à C₄,
R⁶ est un reste phényle, naphtyle ou hétérocyclyle de la série furyle, tétrahydrofuryle, benzofuryle, dihydrobenzofuryle, dioxolanyle, dioxanyle, benzodioxanyle, pyrrolyle, pyrazolyle, imidazolyle, benzimidazolyle, triazolyle, oxazolyle, isoxazolyle, benzoxazolyle, thiazolyle, isothiazolyle, benzothiazolyle, pyridinyle, quinolinyle, isoquinolinyle, pyrimidinyle, pyrazinyle, pyridazinyle, quinazolinyle, quinoxalinyle chacun éventuellement substitué par un radical nitro, cyano, carboxy, carbamoyle, thiocarbamoyle, sulfo, chlorosulfonyle, aminosulfonyle, halogéno, alkyle en C₁ à C₄, halogénalkyle en C₁ à C₄, alkoxy en C₁ à C₄, halogénalkoxy en C₁ à C₄, alkylthio en C₁ à C₄, halogénalkylthio en C₁ à C₄, alkylsulfinyle en C₁ à C₄, halogénalkylsulfinyle en C₁ à C₄, alkylsulfonyle en C₁ à C₄, halogénalkylsulfonyle en C₁ à C₄ ou (alkoxy en C₁ à C₄)-carbonyle, et
R⁷ représente l'hydrogène, un reste alkyle, alkoxy, alkylthio, alkylamino ou dialkylamino ayant chacun 1 à 6 atomes de carbone dans les groupes alkyle et portant chacun un substituant cyano, halogéno ou alkoxy en C₁ à C₄, un reste alcényle ou alcynyle ayant chacun 2 à 6 atomes de carbone et portant chacun le cas échéant un substituant cyano ou halogéno, un reste cyclo-alkyle ou cyclo-alkylalkyle ayant chacun 3 à 6 atomes de carbone dans les groupes cyclo-alkyle et le cas échéant 1 à 4 atomes de carbone dans la partie alkyle et portant chacun le cas échéant un substituant cyano, halogéno ou alkyle en C₁ à C₄, ou bien un reste phényle portant éventuellement un substituant nitro, cyano, carboxy, carbamoyle, thiocarbamoyle, sulfo, chlorosulfonyle, aminosulfonyle, halogéno, alkyle en C₁ à C₄, halogénalkyle en C₁ à C₄, alkoxy en C₁ à C₄, halogénalkoxy en C₁ à C₄, alkylthio en C₁ à C₄, halogénalkylthio en C₁ à C₄, alkysulfinyle en C₁ à C₄, halogénalkylsulfinyle en C₁ à C₄, alkylsulfonyle en C₁ à C₄, halogénalkylsulfonyle en C₁ à C₄ ou (alkoxy en C₁ à C₄,)-carbonyle.

2. Phényluraciles substitués de formule générale (I) suivant la revendication 1, **caractérisés en ce que**
n représente les nombres 0, 1, ou 2,
Q représente O, S, SO, SO₂, NH ou N(CH₃),
R¹ est l'hydrogène, un groupe amino ou un reste méthyle, éthyle, n-propyle ou isopropyle dont chacun est éventuellement substitué par un radical cyano, fluoro, chloro, méthoxy ou éthoxy,
R² est un groupe carboxy, cyano, carbamoyle, thiocarbamoyle ou un reste méthyle, éthyle, n-propyle, isopropyle, méthoxycarbonyle, éthoxycarbonyle, n-propoxycarbonyle ou isopropoxycarbonyle portant chacun le cas échéant un substituant cyano, fluoro, chloro, méthoxy ou éthoxy,
R³ représente l'hydrogène, le fluor, le chlore, le brome ou un reste méthyle, éthyle, n-propyle ou isopropyle chacun éventuellement substitué par du fluor et/ou du chlore,
R⁴ est un groupe amino ou l'un des groupements suivants -NH-CO-R⁷ ou -N(CO-R⁷)₂,
R⁵ est un groupe nitro, amino, hydroxy, mercapto, carboxy, cyano, carbamoyle, thiocarbamoyle, sulfo, chlorosulfonyle, aminosulfonyle, le fluor, le chlore, le brome ou un reste méthyle, éthyle, n-propyle, isopropyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, méthylamino, éthylamino, n-propylamino, isopropylamino, acétyle, propionyle, méthoxycarbonyle, éthoxycarbonyle, méthylaminocarbonyle, éthylaminocarbonyle, acétylamino, propionylamino, méthoxycarbonylamino, éthoxycarbonylamino, méthylsulfonylamino, éthylsulfonylamino, bis-méthylsulfonylamino ou bis-éthylsulfonylamino portant chacun le cas échéant un substituant cyano, fluoro, chloro, méthoxy ou éthoxy,
R⁶ est un reste phényle, naphtyle ou hétérocyclyle de la série furyle, tétrahydrofuryle, . benzofuryle, dihydrobenzofuryle, dioxolanyle, dioxanyle, benzodioxanyle, pyrrolyle, pyrazolyle, imidazolyle, benzimidazolyle, triazolyle, oxazolyle, isoxazolyle, benzoxazolyle, thiazolyle, isothiazolyle, benzothiazolyle, pyridinyle, quinolinyle, isoquinolinyle, pyrimidinyle, pyrazinyle, pyridazinyle, quinazolinyle, quinoxalinyle portant chacun le cas échéant un substituant nitro, cyano, carboxy, carbamoyle, thiocarbamoyle, sulfo, chlorosulfonyle, aminosulfonyle, halogéno, alkyle en C₁ à C₄, halogénalkyle en C₁ à C₄, alkoxy en C₁ à C₄, halogénalkoxy en C₁ à C₄, alkylthio en C₁ à C₄, halogénalkylthio en C₁ à C₄, alkylsulfinyle en C₁ à C₄, halogénalkylsulfinyle en C₁ à C₄, alkylsulfonyle en C₁ à C₄, halogénalkylsulfonyle en C₁ à C₄ ou (alkoxy en C₁ à C₄)-carbonyle,
R⁷ représente l'hydrogène, un reste alkyle, alkoxy, alkylthio, alkylamino ou dialkylamino ayant chacun 1 à 6 atomes de carbone dans les groupes alkyle et portant chacun éventuellement un substituant cyano, halogéno ou alkoxy en C₁ à C₄, un reste alcényle ou alcynyle ayant chacun 2 à 6 atomes de carbone et portant chacun le cas échéant un substituant cyano ou halogéno, un reste cyclo-alkyle ou cyclo-alkylalkyle ayant chacun 3 à 6 atomes de carbone dans les groupes cyclo-alkyle et le cas échéant 1 à 4 atomes de carbone dans la partie alkyle et portant.chacun le cas échéant un substituant cyano, halogéno ou alkyle en C₁ à C₄, ou bien un reste phényle portant éventuellement un substituant nitro, cyano, carboxy, carbamoyle, thiocarbamoyle, sulfo, chlorosulfonyle, aminosulfonyle, halogéno, alkyle en C₁ à C₄, halogénalkyle en C₁ à C₄, alkoxy en C₁ à C₄, halogénalkoxy en C₁ à C₄, alkylthio en C₁ à C₄, halogénalkylthio en C₁ à C₄, alkylsulfinyle en C₁ à C₄, halogénalkylsulfinyle en C₁ à C₄, alkylsulfonyle en C₁ à C₄, halogénalkylsulfonyle en C₁ à C₄ ou (alkoxy en C₁ à C₄,)-carbonyle.

3. Phényluraciles substitués de formule générale (I) suivant la revendication 1, **caractérisés en ce que**
R¹ représente l'hydrogène.

4. Phényluraciles substitués de formule générale (I) suivant la revendication 1, **caractérisés en ce que**
R⁴ est un groupe amino.

5. Procédé de production de phényluraciles substitués de formule générale (I) suivant l'une des revendications 1 à 4, **caractérisé par** la réaction
(a) d'esters d'acides amino-alcénoïques de formule générale (II) 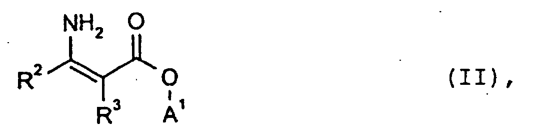 dans laquelle
R² et R³ ont les définitions indiquées dans la revendication 1 ou 2 et
A¹ est un reste alkyle en C₁ à C₄, phényle ou benzyle, avec des phényluréthanes (phénylcarbamates) substitués de formule générale (III) 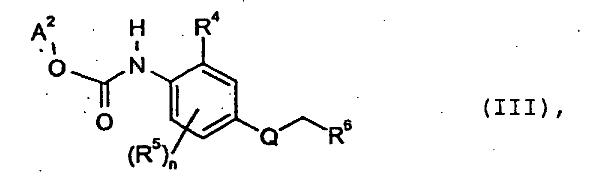 dans laquelle
n, Q, R⁴, R⁵ et R⁶ ont les définitions indiquées dans la revendication 1 ou 2 et
A² est un reste alkyle en C₁ à C₄, phényle ou benzyle, éventuellement en présence d'un auxiliaire de réaction et, le cas échéant, en présence d'un diluant,
ou par réaction
(b) de phényluraciles substitués de formule générale (Ia), 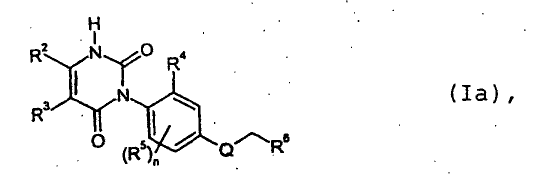 dans laquelle
n, Q, R², R³, R⁴, R⁵ et R⁶ ont les définitions indiquées dans la revendication 1 ou 2,
avec le 1-amino-oxy-2,4-dinitro-benzène ou avec des agents d'alkylation de formule générale (IV)
X¹-A³ (IV)
dans laquelle
A³ est un reste alkyle ayant 1 à 4 atomes de carbone éventuellement substitué par un radical cyano, halogéno ou alkoxy en C₁ à C₄ et
X¹ représente le chlore, le brome, l'iode ou le groupement méthylsulfonyloxy ou éthylsulfonyloxy,
éventuellement en présence d'un auxiliaire de réaction et, le cas échéant, en présence d'un diluant
ou par réaction
(c) de phényluraciles substitués de formule générale (Ib), 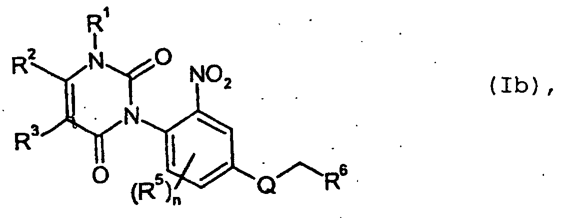 dans laquelle
n, Q, R¹, R², R³, R⁵ et R⁶ ont les définitions indiquées dans la revendication 1 ou 2,
avec des agents d'hydrogénation, éventuellement en présence d'auxiliaires de réaction et, le cas échéant, en présence de diluants ou par réaction
(d) de phényluraciles substitués de formule générale (Ic), 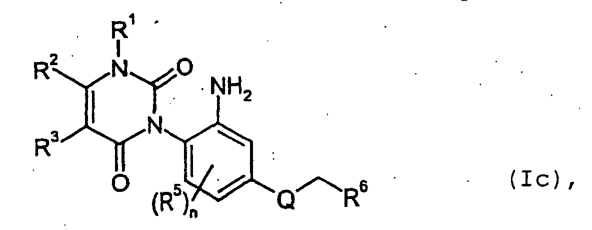 dans laquelle
n, Q, R¹, R², R³, R⁵ et R⁶ ont les définitions indiquées dans la revendication 1 ou 2,
avec des agents d'acylation de formule générale (V)
X²-CO-R⁷ (V)
dans laquelle
R⁷ a la définition indiquée dans la revendication 1 ou 2 et
X² représente le fluor, le chlore ou le brome,
éventuellement en présence d'auxiliaires de réaction et, le cas échéant, en présence de diluants.

6. Utilisation d'au moins un phényluracile substitué suivant l'une des revendications 1 à 4 pour la lutte contre des plantes indésirables.

7. Composition herbicide, **caractérisée par** la teneur en au moins un phényluracile substitué suivant l'une des revendications 1 à 4.
